# EUROPEAN PATENT SPECIFICATION

(11) **EP 3 579 696 B1**
(45) Date of publication and mention of the grant of the patent: **28.06.2023**
(21) Application number: 18709829.8
(22) Date of filing: 08.02.2018
(51) Int. Cl.: A01N 43/72, A01P 5/00, A01N 47/18, A01N 43/90, A61P 33/10

(54) **IN VIVO MODEL FOR PARASITIC WORM INFECTION AND METHODS FOR EVALUATING ANTIPARASITIC COMPOUNDS, INCLUDING COMPOUNDS ACTIVE AGAINST CANINE HEARTWORM**
IN-VIVO-MODELL FÜR PARASITISCHE WURM-INFEKTION UND VERFAHREN ZUR BEWERTUNG ANTIPARASITISCHER VERBINDUNGEN, EINSCHLIESSLICH VERBINDUNGEN MIT AKTIVITÄT GEGEN HUNDE-HERZWURM
MODÈLE IN VIVO POUR UNE INFECTION PAR DES VERS PARASITAIRES ET PROCÉDÉS D'ÉVALUATION DE COMPOSÉS ANTIPARASITIQUES, Y COMPRIS DES COMPOSÉS ACTIFS CONTRE LE DIROFILARIA DES CHIENS

(30) Priority: 08.02.2017 US 201762456510 P
(43) Date of publication of application: 18.12.2019
(73) Proprietor: Boehringer Ingelheim Animal Health USA Inc., Duluth, GA 30096 (US); Thomas Jefferson University, Philadelphia, PA 19107 (US)
(72) Inventor: ABRAHAM, David, Wynnewood, PA 19096 (US); HESS, Jessica, Willow Grove, PA 19090 (US); BONDESEN, Brenda, A., Duluth, GA 30096 (US); HARRINGTON, John, Matthew, Athens, GA 30607 (US)
(74) Representative: D Young & Co LLP
(86) International application number: PCT/US2018/017398
(87) International publication number: WO 2018/148392

(56) References cited:
- WO-A1-2016/161369
- V. KUMARASWAMI: "Ivermectin for the Treatment of Wuchereria bancrofti Filariasis : Efficacy and Adverse Reactions", JAMA THE JOURNAL OF THE AMERICAN MEDICAL ASSOCIATION, vol. 259, no. 21, 3 June 1988 (1988-06-03) , page 3150, XP55465710, US ISSN: 0098-7484, DOI: 10.1001/jama.1988.03720210040026
- DOMINIQUE RICHARD-LENOBLE ET AL: "Ivermectin and filariasis", FUNDAMENTAL & CLINICAL PHARMACOLOGY., vol. 17, no. 2, 1 April 2003 (2003-04-01), pages 199-203, XP55465714, FR ISSN: 0767-3981, DOI: 10.1046/j.1472-8206.2003.00170.x
- M T Suderman ET AL: "Efficacy of ivermectin against Dirofilaria immitis microfilariae in naturally infected dogs.", Am J Vet Res., 1 May 1984 (1984-05-01), pages 1031-1032, XP55465753, Retrieved from the Internet: URL:http://www.ncbi.nlm.nih.gov/pubmed/654 7279 [retrieved on 2018-04-10]
- BYRON L. BLAGBURN ET AL: "Efficacy of four commercially available heartworm preventive products against the JYD-34 laboratory strain of Dirofilaria immitis", PARASITES & VECTORS, vol. 52, no. Suppl 1, 5 April 2016 (2016-04-05), page 2040, XP55281230, DOI: 10.1186/s13071-016-1476-7
- T. J. NOLAN ET AL: "Strongyloides stercoralis: The First Rodent Model for Uncomplicated and Hyperinfective Strongyloidiasis, the Mongolian Gerbil (Meriones unguiculatus)", JOURNAL OF INFECTIOUS DISEASES. JID, vol. 168, no. 6, 1 December 1993 (1993-12-01), pages 1479-1484, XP55465796, CHICAGO, IL. ISSN: 0022-1899, DOI: 10.1093/infdis/168.6.1479
- ANNA CRISFORD ET AL: "The Cyclooctadepsipeptide Anthelmintic Emodepside Differentially Modulates Nematode, Insect and Human Calcium-Activated Potassium (SLO) Channel Alpha Subunits", PLOS NEGLECTED TROPICAL DISEASES, vol. 9, no. 10, 5 October 2015 (2015-10-05), page e0004062, XP55465725, DOI: 10.1371/journal.pntd.0004062
- K.R. BROWN ET AL: "Ivermectin: effectiveness in lymphatic filariasis", PARASITOLOGY, vol. 121, no. S1, 15 October 2000 (2000-10-15), page S133, XP55465713, GB ISSN: 0031-1820, DOI: 10.1017/S0031182000006570
- S. K. TAGBOTO ET AL: "Onchocerca volvulus and O. lienalis: the microfilaricidal activity of moxidectin compared with that of ivermectin in vitro and in vivo", ANNALS OF TROPICAL MEDICINE AND PARASITOLOGY., vol. 90, no. 5, 15 January 1996 (1996-01-15), pages 497-505, XP55465817, GB ISSN: 0003-4983, DOI: 10.1080/00034983.1996.11813075
- Silvia Puspitasari ET AL: "Effectiveness of Ivermectin and Albendazole against Haemonchus contortus in Sheep in West Java, Indonesia", Tropical life sciences research, 1 February 2016 (2016-02-01), pages 135-144, XP55465733, Malaysia Retrieved from the Internet: URL:https://www.ncbi.nlm.nih.gov/pmc/artic les/PMC4807958/pdf/tlsr-27-1-135.pdf
- MADS BJELKE PETERSEN ET AL: "A new in vitro assay of benzimidazole activity against adult Oesophagostomum dentatum", INTERNATIONAL JOURNAL OF PARASITOLOGY, vol. 27, no. 11, 1 November 1997 (1997-11-01), pages 1333-1339, XP55465737, GB ISSN: 0020-7519, DOI: 10.1016/S0020-7519(97)00104-5
- SANDRA BONNE-ANNÉE ET AL: "Innate and adaptive immunity to the nematodein a mouse model", IMMUNOLOGIC RESEARCH, HUMANA PRESS INC, NEW YORK, vol. 51, no. 2 - 3, 19 November 2011 (2011-11-19), pages 205-214, XP019990829, ISSN: 1559-0755, DOI: 10.1007/S12026-011-8258-2
- F K Nelson ET AL: "The immunodeficient scid mouse as a model for human lymphatic filariasis", The Journal of experimental medicine, 1 March 1991 (1991-03-01), pages 659-663, XP55465769, UNITED STATES DOI: 10.1084/jem.173.3.659 Retrieved from the Internet: URL:http://images.biomedsearch.com/1997651 /je1733659.pdf?AWSAccessKeyId=AKIAIBOKHYOL P4MBMRGQ&Expires=1523491200&Signature=5AyH 3wxVdxCtWF1sPfr3Cf0t2V8=
- HEIDI L SMITH ET AL: "Development of a Serum-Free System for the in Vitro Cultivation of Brugia malayi Infective-Stage Larvae", EXPERIMENTAL PARASITOLOGY, vol. 95, no. 4, 1 August 2000 (2000-08-01) , pages 253-264, XP55465768, US ISSN: 0014-4894, DOI: 10.1006/expr.2000.4531
- YOICHI ET AL: "The absence of resistance in congenitally athymic nude mice toward infection with the intestinal nematode, Trichuris muris: Resistance restored by lymphoid cell transfer", INTERNATIONAL JOURNAL OF PARASITOLOGY, PERGAMON PRESS, GB, vol. 21, no. 1, 1 February 1991 (1991-02-01), pages 65-69, XP023652187, ISSN: 0020-7519, DOI: 10.1016/0020-7519(91)90121-M [retrieved on 1991-02-01]
- J Andreassen ET AL: "Hymenolepis diminuta infections in congenitally athymic (nude) mice: worm kinetics and intestinal histopathology", Immunology, 1 January 1978 (1978-01-01), pages 105-113, XP55465677, ENGLAND Retrieved from the Internet: URL:https://www.ncbi.nlm.nih.gov/pmc/artic les/PMC1457348/pdf/immunology00276-0111.pd f
- H L Rotman ET AL: "Strongyloides stercoralis: complete life cycle in SCID mice", Experimental Parasitology, 1 January 1995 (1995-01-01), pages 136-139, XP55465684, U.S.A. Retrieved from the Internet: URL:https://ac.els-cdn.com/S00144894857110 10/1-s2.0-S0014489485711010-main.pdf?_tid= dfd0cfe0-3e6e-4fbc-b703-f73263b55292&acdna t=1523287177_fd502af0e07223e7d918059ddf453 722 [retrieved on 2018-04-09]
- TIAGO MENDES ET AL: "Strongyloidiasis Current Status with Emphasis in Diagnosis and Drug Research", JOURNAL OF PARASITOLOGY RESEARCH, vol. 2017, 1 January 2017 (2017-01-01), pages 1-13, XP55465680, ISSN: 2090-0023, DOI: 10.1155/2017/5056314
- DANIEL KULKE ET AL: "Efficacy of Cyclooctadepsipeptides and Aminophenylamidines against Larval, Immature and Mature Adult Stages of a Parasitologically Characterized Trichurosis Model in Mice", PLOS NEGLECTED TROPICAL DISEASES, vol. 8, no. 2, 1 February 2014 (2014-02-01), page e2698, XP55465765, DOI: 10.1371/journal.pntd.0002698
- P D Walzer ET AL: "Immunodeficient and immunosuppressed mice as models to test anti-Pneumocystis carinii drugs", Antimicrobial Agents and Chemotherapy, 1 February 1997 (1997-02-01), pages 251-258, XP55465856, UNITED STATES Retrieved from the Internet: URL:http://aac.asm.org/content/41/2/251.fu ll.pdf
- ADAMS ET AL: "Infection of immunosuppressed mice with the abomasal nematode parasite of ruminants, Haemonchus contortus", INTERNATIONAL JOURNAL OF PARASITOLOGY, PERGAMON PRESS, GB, vol. 20, no. 5, 1 August 1990 (1990-08-01) , pages 631-636, XP023662680, ISSN: 0020-7519, DOI: 10.1016/0020-7519(90)90121-3 [retrieved on 1990-08-01]
- Rebecca Fankhauser ET AL: "Use of Rodent Models in the Discovery of Novel Anthelmintics : Targets, Screens, Drugs and Vaccines" In: "Parasitic Helminths", 18 July 2012 (2012-07-18), Wiley-VCH Verlag GmbH & Co. KGaA, Weinheim, Germany, XP55465800, ISBN: 978-3-527-33059-1 pages 181-199, DOI: 10.1002/9783527652969.ch11, abstract table 11.2
- JENNIFER KEISER ET AL: "Strongyloides ratti: In Vitro and In Vivo Activity of Tribendimidine", PLOS NEGLECTED TROPICAL DISEASES, vol. 2, no. 1, 23 January 2008 (2008-01-23), page e136, XP055466381, DOI: 10.1371/journal.pntd.0000136
- JENNIFER KEISER ET AL: "Evaluation of an FDA approved library against laboratory models of human intestinal nematode infections", PARASITES & VECTORS, vol. 9, no. 1, 1 July 2016 (2016-07-01), XP055466538, DOI: 10.1186/s13071-016-1616-0
- SHULTZ L D ET AL: "Humanized mice in translational biomedical research", THE JOURNAL OF IMMUNOLOGY, NATURE PUB. GROUP, GB, vol. 7, no. 2, 1 February 2007 (2007-02-01), pages 118-130, XP002493022, ISSN: 1474-1733, DOI: 10.1038/NRI2017
- Hanna Sjoberg ET AL: "Development of murine models of Ioiasis to assess microfilaricidal activity of pre-clinical candidate anti-filarial drugs", BSP Spring Meeting 2016, 11 April 2016 (2016-04-11), pages 1-1, XP055466703, London, U.K. Retrieved from the Internet: URL:https://www.myeventflo.com/event-lectu re.asp?lectID=10186 [retrieved on 2018-04-12]

## Description

### FIELD OF THE INVENTION

The invention relates to the modeling of parasitic worm infections using immunocompromised rodents, with or without engrafted immune cells from a definitive host of the parasitic worm. The invention further relates to the use of such model systems to screen and evaluate the efficacy of antiparasitic agents.

### BACKGROUND OF THE INVENTION

Filariasis is a parasitic disease caused by thread-like filarial nematodes or roundworms. Filariasis is transmitted via insect bites, and infective filarial larvae may be introduced into animals, including dogs and humans, via bites of blood sucking insects including mosquitoes or flies. In dogs, cardiopulmonary dirofilariasis, (i.e., heartworm (HW) disease) is caused by *Dirofilaria immitis* (*D. immitis*) and *Dirofilaria repens* (*D. repens*) nematodes. In the case of *D. repens,* adults are subcutaneous, so while they don't cause heartworm disease *per se,* they do cause disease/pathology and are prevented with ivermectin (IVM) doses comparable to those used to prevent HW. Dirofilariasis is a severe vector-borne helminthiasis of dogs and cats worldwide. It is transmitted by Culicidae (i.e., mosquito) bites and caused by the development of *D. immitis* in the pulmonary arteries and the heart. Clinically, it leads to irreversible cardiac insufficiency that can result in the death of the animal. Treatment is difficult and prevention is critical for dirofilariasis control. In humans, clinically significant filarial nematodes include *Wuchereria bancrofti, Brugia malayi, Brugia timori, Onchocerca volvulus* and *Mansonella* species. *W. bancrofti* and *B. malayi* infections can develop into lymphatic filariasis, often presenting as hydrocoele and/or lymphedema, and (in extreme cases), elephantiasis. *O*. *volvulus* infections can evolve into *onchocerciasis,* the visual impairment giving the disease its common name River Blindness. The nematodes are host to obligate intracellular bacteria of the genus *Wolbachia.* These endobacteria are essential for embryogenesis, larval development and adult worm survival. As such, an alternative effort aiming to eliminate the nematodes includes removal of the bacteria with antibacterial agents.

The development of these types of parasitic worms, particularly Dirofilaria, is often highly restricted to the definitive (mammalian) host, precluding the development of intermediate animal/rodent models. The ramifications of this are apparent in the lack of new pharmaceutical agents to combat these pathogens. For example, macrocyclic lactones (MLs) were introduced more than 25 years ago, yet they remain the only chemical class approved for HW disease prevention. For most investigators, canine studies are simply too long-at least six months-and expensive to justify exploratory studies outside this proven class of compounds. Therefore, an intermediate model of filarial infection in rodents could open the discovery floodgates, allowing researchers to rapidly screen and evaluate novel parasiticidal agents. As of the filing of this application, no one has successfully modeled such filarial infections outside the definitive host.

H. L. Rotman et al, Experimental Parasitology, 81, 136-139, 1995 describes the life cycle of *Strongyloides stercoralis* in SCID mice.

T. Mendes et al, "Strongyloidiasis Current Status with Emphasis in Diagnosis and Drug Research", Journal Of Parasitology Research, (20170101), vol. 2017, pages 1 - 13, is a review article which discusses the current status of Strongyloidiasis diagnosis and drug research.

D. Kulke et al, "Efficacy of Cyclooctadepsipeptides and Aminophenylamidines against Larval, Immature and Mature Adult Stages of a Parasitologically Characterized Trichurosis Model in Mice", PLOS Neglected Tropical Diseases, (20140201), vol. 8, no. 2, page e2698, discloses orally infecting C57BL/10 ScSnOlaHsd mice with eggs of Trichus muris.

J. Keiser et al, "Evaluation of an FDA approved library against laboratory models of human intestinal nematode infections", Parasites & Vectors, 2016, vol. 9, no. 1 discloses using animal models of human intestinal nematode infections and testing the activity of an FDA approved drug library.

L. D. Shultz et al, "Humanized mice in translational biomedical research", The Journal Of Immunology, 2007, vol. 7, no. 2, pages 118 - 130 is a review article discussing the use of humanized mice in translation biomedical research.

H. Sjoberg et al, "Development of murine models of Ioiasis to assess microfilaricidal activity of pre-clinical candidate anti-filarial drugs", BSP Spring Meeting 2016, London, U.K., URL: https://www.myeventflo.com/event-lecture.asp?lectID=10186, is a conference abstract, which discloses the development of mouse models of Ioiasis with the aim of evaluating them as in vivo microfilaricide drug screens.

### SUMMARY OF THE INVENTION

Applicants have found, quite unexpectedly, that NOD scid gamma (NOD.Cg-Prkdcscid Il2rgtm1Wjl/SzJ; hereafter, "NSG") immunodeficient mice support complete infection and development of certain parasitic worms, including canine HW. These mice support complete infection and development of filarial parasitic worms, such as those in Table 1, as well as other parasitic nematodes, such as *Haemonchus contortus.* Additionally, Applicants discovered that NRG (*NOD-Rag1^{null} IL2rg^{null},* NOD rag gamma), a similarly immunodeficient mouse, is susceptible to HW, whereas other immunodeficient mice are resistant.

**Table 1. Immunocompromised mouse unexpectedly supports complete infection for some nematode species, but not others.**

| **Parasite** | **Definitive Host** | **Complete Infection in Normal Mouse?** | **Complete Infection in NSG Mouse?** |
|---|---|---|---|
| *Dirofilaria immitis* (Heartworm Disease) | Canine | No | Yes |
| *Onchocerca volvulus* (River Blindness) | Human | No | No |
| *Strongyloides stercoralis* | Human, Canine | No | Yes |
| *Haemonchus contortus* | Sheep, goats | No | Yes |

Accordingly, in a first aspect, the invention provides a method of determining the effectiveness of a compound or combination of compounds for treating or preventing a parasitic worm infection, comprising:
(a) infecting an immunocompromised rodent with a specific number of parasitic worm larvae;
(b) treating the infected rodent with the compound or combination of compounds to be tested; and
(c) necropsying the rodent and determining the number of parasitic worms, optionally adult parasitic worms, present in a rodent tissue;
wherein the rodent is an NOD scid IL2 receptor gamma chain knockout mouse (NSG) (NOD-SCID-IL2Ry -/-) or an NRG (NOD-Rag1^{null}IL2rg^{null}) immunodeficient mouse; and wherein the parasitic worm is a nematode and the parasitic worm is *Dirofilaria immitis, Strongyloides stercoralis* or *Haemonchus contortus.*

Described is an immunocompromised rodent model for supporting the infection and development of parasitic worms (e.g., nematode parasites such as filarial parasites) outside of their definitive hosts. Such immunocompromised rodent models can support the complete infection and development of filarial parasites (e.g., to the stage of adult worms) outside of their definitive hosts. Such immunocompromised rodent models can be used in methods for modeling complete infection of a parasitic worm outside of the worm's definitive host, methods of determining the effectiveness of a compound for treating or preventing a parasitic worm infection, methods of drug screening for drugs with efficacy in treating or preventing a parasitic worm infection, or methods of characterizing changes in sensitivity to a parasiticidal agent during parasitic worm infection (e.g., a filaricidal agent during filarial worm infection).

In a second aspect, the invention provides a method of determining the number of parasitic worms in one or more isolated tissue sample(s) of an immunocompromised rodent, preferably necropsied immunocompromised rodent, infected with a specific number of parasitic worm larvae and treated with a compound or combination of compounds to be tested, wherein the method comprises determining the number of parasitic worms, optionally adult parasitic worms, present in the isolated tissue sample(s); wherein the parasitic worm is a nematode and the parasitic worm is *Dirofilaria immitis, Strongyloides stercoralis* or *Haemonchus contortus* and wherein the rodent is an NOD scid IL2 receptor gamma chain knockout mouse (NSG) (NOD-SCID-IL2Ry -/-) or an NRG (NOD-Rag1^{null}IL2rg^{null}) immunodeficient mouse.

In a third aspect, the invention provides one or more filaricidal compound(s) identified by the method of the first or second aspects above, selected from the group consisting of 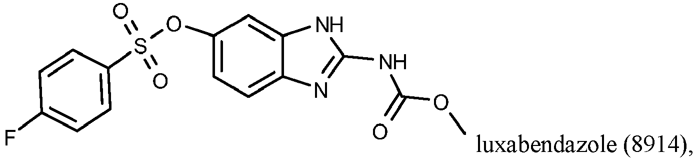 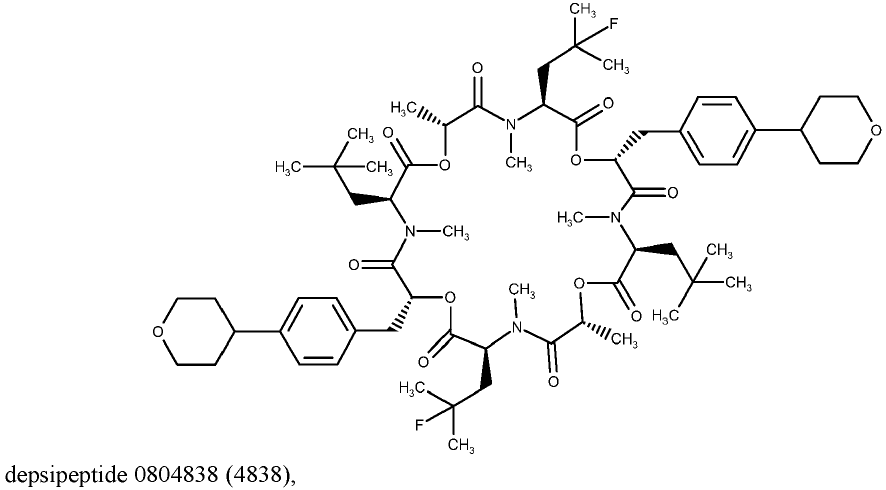 and 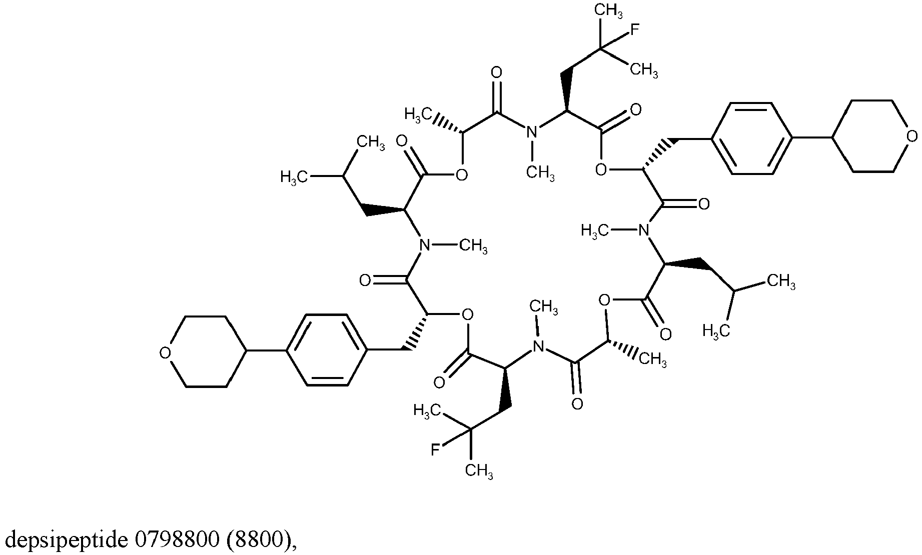 for use in a method of treating filariasis in a subject in need thereof, wherein the filariasis is dirofilariasis.

In a fourth aspect, the invention provides an immunocompromised rodent infected with one or more adult parasitic worms, wherein the parasitic worm is a nematode and wherein the parasitic worm is *Dirofilaria immitis, Strongyloides stercoralis* or *Haemonchus contortus* and the rodent is an NOD scid IL2 receptor gamma chain knockout mouse (NSG) (NOD-SCID-IL2Ry -/-) or an NRG (NOD-Rag1^{null}IL2rg^{null}) immunodeficient mouse.

In a fifth aspect, the invention provides the use of an immunocompromised rodent of the fourth aspect above for modeling complete infection of a parasitic worm outside of the worm's definitive host or for characterizing changes in sensitivity to a parasiticidal or filaricidal agent during parasitic or filarial worm infection.

In a sixth aspect, the invention provides the use of an immunocompromised rodent for modeling complete infection of a parasitic worm outside of the worm's definitive host, wherein the rodent is an NOD scid IL2 receptor gamma chain knockout mouse (NSG) (NOD-SCID-IL2Ry -/-) or an NRG (NOD-Rag1^{null}IL2rg^{null}) immunodeficient mouse and the parasitic worm is a nematode and the parasitic worm is *Dirofilaria immitis, Strongyloides stercoralis* or *Haemonchus contortus.*

### BRIEF DESCRIPTION OF THE DRAWINGS

The following detailed description may best be understood in conjunction with the accompanying drawings, in which:
FIG. 1 is a graph showing the growth of *D. immitis* recovered from NSG mice at the indicated times post-infection with 100 L3 per NSG mouse.
FIG. 2 is a graph showing the normalized percent larva recovery (day 42/6 weeks) after *D. immitis (Di)*-infected NSG mice were dosed with 1 mg/kg emodepside or 3 mg/kg ivermectin, at Days 1, 15, and 30 post-infection.
FIG. 3 is a graph showing the lengths of *Di* recovered at Days 41 and 43, post-infection, from NSG mice that were administered Control, EMO, or 8800 on Day 30, post-infection.
FIG. 4 is a graph showing the lengths of *Di* recovered at Days 83 and 84. Control, EMO, or 8800 treatments was administered to the Di-infected mice on Days 30 and 60.
FIG. 5 is a graph showing the lengths of *Di* recovered at Days 41 to 43. Control and various concentrations of 8800, EMO, or IVM was administered to the Di-infected mice on Days 30 and 60.
FIG. 6 is a graph showing length of *Di* recovered at Days 41 to 43. Control, EMO, 8800, 4838, 8914, or 6557 was administered to the *Di*-infected mice on Days 1, 15, and 30 post-infection.
FIG. 7 is a graph showing the lengths of *Di* recovered at Days 41 to 43. Control, EMO, 8800, 4838, or IVM was administered to the *Di*-infected mice on Days 1, 15, and 30 post-infection.
FIG. 8 is a graph showing the lengths of *Di* recovered at Days 41 to 42. Control, EMO, 8800, or 4838 was administered to the *Di*-infected mice on Days 1, 15, and 30 post-infection.
FIG. 9 is a graph showing the lengths of *Di* recovered at Days 41 to 42. Control, EMO, 8800, or 4838 was administered to the *Di*-infected mice on Days 1, 15, and 30 post-infection.
FIG. 10 is a graph showing the lengths of *Di* recovered at Days 40 to 42. Control, EMO, 8800, or 4838 was administered to the *Di*-infected mice on Days 1, 15, and 30 post-infection.
FIG. 11 is a graph showing the lengths of *Di* recovered at Days 41 to 42. Control, IVM, 8800, or 4838 was administered to the *Di*-infected mice on Days 1, 15, and 30 post-infection.
FIG. 12 is a graph showing the lengths of *Di* recovered at Days 41 to 42. Control or IVM was administered to the *Di*-infected mice on Days 1, 15, and 30 post-infection. The Missouri and JYD strains of *Di* were tested.
FIG. 13 shows worm recovery locations in *Di*-infected mice administered control or IVM on Days 1, 15, and 30 post-infection.
FIG. 14 is a graph showing the lengths of *Di* recovered at Days 41 to 42 post-infection from C57BL/6, SCID, NOD, NOD/SCID, NSG, and NRG mice.
FIG. 15 is a graph showing the lengths of *Di* recovered at Days 41 to 42. Control or 4838 was administered to the *Di*-infected mice on Days 1, 15, and 30 post-infection.
FIG. 16 is a graph showing the lengths of *Di* recovered at Days 41 to 42. Control, 3615, 5010, 7823, 2677, or 8800 was administered to the *Di*-infected mice on Days 1, 15, and 30 post-infection.
FIG. 17 is a graph showing the lengths of *Di* recovered at Days 40 to 42. Control or IVM was administered to the *Di*-infected mice on Days 1, 15, and 30 post-infection.
FIG. 18 is a graph showing the lengths of *Di* recovered at Days 40 to 42. Control or 7823 was administered to the *Di*-infected mice on Days 1, 15, and 30 post-infection.
FIG. 19 is a graph showing the lengths of *Di* recovered at Days 40 to 42. Control or IVM was administered to the *Di*-infected mice on Days 1, 15, and 30 post-infection.
FIG. 20 is a graph showing the lengths of *Di* recovered at Days 40 to 42. Control or IVM was administered to the *Di*-infected mice on Days 1, 15, and 30 post-infection.
FIG. 21 is a graph showing the lengths of *Di* recovered at Days 40 to 42 post-infection from mice with engrafted canine CD34+ cord blood cells.
FIG. 22 is a graph showing the number of worms recovered in control and MPA-treated NSG mice infected with 500 L3 *H. contortus.*
FIG. 23 is a graph showing the number of worms recovered in control and MPA-treated NSG mice infected with 1000 L3 *H. contortus.*

### DETAILED DESCRIPTION

The invention is based upon the surprising discovery that immunocompromised rodents, with or without engrafted immune cells from a different species (e.g., the species of a definitive host of a parasitic worm), may be used to model filarial parasite infections and infections by other parasitic worms such as nematode parasites outside of their definitive hosts. Applicants have found, quite unexpectedly, that NSG and NRG immunodeficient mice support infection and development of certain parasitic worms, including canine HW.

NSG and NRG mice are among the most immunocompromised mice described to date, lacking mature T cells, B cells, and natural killer (NK) cells. Moreover, NSG mice lack several cytokine signaling pathways, and they have many defects in innate immunity. As indicated in Table 1, Applicants found that NSG and NRG mice exhibit a surprising capacity to harbor parasites that until now have normally only established complete infections in definitive hosts, but have not established complete infections in non-target animals (i.e., non-definitive hosts) having uncompromised immune systems (Table 1). Likewise, Applicants found that NSG mice with engrafted immune cells from the definitive host of a parasitic worm exhibit a surprising capacity to harbor parasites that until now have normally only established complete infections in definitive hosts. As used herein, "complete infection" means that a given early stage larval form (e.g., L3) persists and develops into mature worms (e.g., adult worms). In the case of filarial parasites, the given early stage larval form (e.g., L3) persists and develops into mature worms that may be capable of releasing microfilariae into the bloodstream of the infected/infested host. Microfilariae themselves do not establish infection. Instead, these earliest stage larval forms are taken up from an infected mammal via a bite from a blood-sucking arthropod (e.g., mosquito). Within the vector, microfilariae develop into L3 larvae, which are introduced into subsequent mammalian hosts when an L3-infected vector bites and feeds upon the mammal.

This finding is unexpected, in part, because a skilled person would not have held a reasonable expectation that L3 larvae would persist in a non-definitive host for the observed long (months) periods of time. As such, prior to this disclosure, it could have been the case that the immunocompromised mouse was good for harboring the L3 stage, but only for a limited period of time. Such a model might have been sufficient to test therapeutic agents for their ability to kill L3 larvae. However, much to Applicants' surprise, the L3 larvae developed into L4 and adults when introduced into the immunocompromised rodent. In the case of *Dirofilaria immitis,* for example, this provides an excellent model for canine cardiopulmonary dirofilariasis. Applicants' establishment of this model permits the rapid screening of APIs for their ability to target one or more specific stages of a filarial nematode's live cycle. Applicants' establishment of this model also permits the rapid screening of APIs for their ability to target one or more specific stages of the life cycle of other parasitic worms or nematode parasites. This feature has the obvious benefit of allowing investigators to target parasite life cycle stages that were heretofore extremely expensive and challenging to address because of the requirement for a definitive host. Moreover, Applicants envision that this model will provide an invaluable tool for interrogating the nature of the signals that allow immunocompromised rodents to mimic canine filarial infection and development. Finally, Applicants envision that when microfilariae are introduced into the immunocompromised rodents, they may persist for a period of time, particularly in circulation when introduced via the IV route. Such persistence would allow for the testing of APIs that are specifically active against microfilariae *in vivo.* Likewise, Applicants envision that when infective larvae (e.g., L3) of nematode parasites are introduced into the immunocompromised rodents, they may persist for a period of time and, for example, develop into adult worms, allowing for API testing.

In a first aspect, the invention provides a method of determining the effectiveness of a compound or combination of compounds for treating or preventing a parasitic worm infection, comprising:
(a) infecting an immunocompromised rodent with a specific number of parasitic worm larvae;
(b) treating the infected rodent with the compound or combination of compounds to be tested; and
(c) necropsying the rodent and determining the number of parasitic worms, optionally adult parasitic worms, present in a rodent tissue;
wherein the rodent is an NOD scid IL2 receptor gamma chain knockout mouse (NSG) (NOD-SCID-IL2Ry -/-) or an NRG (NOD-Rag1^{null}IL2rg^{null}) immunodeficient mouse; and wherein the parasitic worm is a nematode and the parasitic worm is *Dirofilaria immitis, Strongyloides stercoralis* or *Haemonchus contortus.*

The immunocompromised rodent model can support the complete infection and development of filarial parasites (e.g., to adult filarial worms) outside of their definitive hosts. Such immunocompromised rodent models can be used in methods for modeling complete infection of a parasitic worm outside of the worm's definitive host, methods of determining the effectiveness of a compound for treating or preventing a parasitic worm infection, methods of drug screening for drugs with efficacy in treating or preventing a parasitic worm infection, or methods of characterizing changes in sensitivity to a parasiticidal agent during parasitic worm infection (e.g., a filaricidal agent during filarial worm infection).

The model comprises infection of a mouse lacking mature or functional T cells, B cells, and natural killer (NK) cells. In some examples, the model comprises infection of a mouse lacking mature T cells, B cells, and natural killer (NK) cells; and, wherein the mouse also lacks several cytokine signaling pathways and comprises many defects in innate immunity. The model is an NSG mouse or an NRG mouse as described elsewhere herein. Also described herein but not part of the present invention are mice that can be genetically engineered to lack one or more or all comparable functional elements that are lacking in an NSG mouse, relative to a corresponding mouse possessing an intact immune system.

NSG (NOD.*Cg-Prkdc^{scid}Il2rg^{tm1Wjl}*/SzJ) is one of the most immunodeficient mouse strains. The Non-Obese Diabetic (NOD) genetic background contains alleles that reduce the function of the innate branch of the immune system. Consequently, macrophages and dendritic cells are defective. *Scid* is a loss-of-function mutation of the *Prkdc* gene that prevents the development of T and B cells. *Prkdc* encodes the catalytic subunit of a DNA dependent protein kinase with a role in resolving the DNA double strand breaks that occur during V(D)J recombination (i.e., via non-homologous end joining (NHEJ)). In the absence of V(D)J recombination, the T cell receptor (TCR) gene in T cells and the immunoglobulin (Ig) gene in B cells are not expressed, and T and B cells cannot mature. Due to the NHEJ defect in *scid* mutants, *scid* mice lack both mature T and B cells. The gamma chain of the interleukin 2 receptor (*Il2rg*) is a common component of the cell surface receptors for six different interleukins (IL-2, IL-4, IL-7, IL-9, IL-15, and IL-21). NSG mice have a complete null mutation (knockout) of this gene. The signaling pathways for these cytokines are blocked in Il2rg knockout mice, although the cytokines themselves are still present. The major consequence of Il2rg deficiency is an absence of functional NK cells, which require IL15 signaling to develop. NSG mice have the following characteristics: mature B cells: absent; mature T cells: absent; dendritic cells: defective; macrophages: defective; natural killer cells: absent; and complement: absent.

NSG and NRG (NOD rag gamma; NOD.Cg*-Rag1^{tm1Mom} Il2rg^{tm1Wjl}*/SzJ; NOD Rag1^{null} Il2rg^{null}) mice are very similar strains. NRG mice substitute the Rag1 knockout mutation for the scid mutation. The Rag1 knockout has a very similar phenotype in the immune system (elimination of T and B cells), but it does not have the *scid* side effect of radiation/chemotherapy sensitivity. NRG mice carry two mutations on the NOD/ShiLtJ genetic background: a targeted knockout mutation in recombination activating gene 1 (*Rag1*)*,* and a complete null allele of the IL2 receptor common gamma chain (*Il2rg*)*.* The *Rag1^{null}* mutation renders the mice Band T cell deficient, and the *IL2rg^{null}* mutation prevents cytokine signaling through multiple receptors, leading to a deficiency in functional NK cells.

Also described herein but not part of the present invention is a model comprising a mammal whose immune system has been genetically engineered to lack comparable functional elements that are lacking in an NSG mouse, relative to a corresponding mouse possessing an intact immune system. Such models can have, for example, one or more or all of the following characteristics: (1) absence of functional T and B cells (i.e., lack of mature T and B cells); (2) absence of functional NK cells; and (3) reduced function of the innate branch of the immune system. Some models lack mature T and B cells. This can be caused, for example, by a loss of function mutation in the *Prkdc* gene, such as the *Scid* mutation. This can also be caused, for example, by a loss of function mutation in the *Rag1* gene. Some models lack functional NK cells. This can be caused, for example, by a loss-of-function mutation in the *Il2rg* gene, which can cause deficient IL 15 signaling. Some models have deficient cytokine production, such as deficient interleukin signaling pathways (e.g., those affected by *Il2rg* knockout, such as II,15 signaling). Some models have reduced function of the innate branch of the immune system. For example, macrophages and/or dendritic cells can be defective in some models. This can be caused, for example, by the NOD genetic background. Some models lack mature T and B cells and lack functional NK cells. Some models lack mature T and B cells and have reduced function of the innate branch of the immune system. Some models lack functional NK cells and have reduced function of the innate branch of the immune system. Some models lack mature T and B cells, lack functional NK cells, and have reduced function of the innate branch of the immune system.

In some embodiments, the mammal may comprise engrafted immune cells from another species, such as engrafted immune cells from a definitive host of a particular parasitic worm. In some embodiments, the mammal may comprise engrafted hematopoietic stem cells. In some embodiments, the mammal may comprise engrafted mature immune cells. In some embodiments, the mammal may comprise engrafted canine stem cells. In other embodiments, the mammal may comprise engrafted canine mature immune cells. Such mammals may lack functional NK cells and/or lack mature T and B cells (e.g., have the *scid* mutation). In some cases, the mammal can be irradiated prior to engraftment. The mammal is a mouse. The severe immunodeficiency of NSG and NRG mice allows them to be caninized by engraftment of canine CD34+ hematopoietic stem cells (HSCs) and canine derived xenografts at high efficiency. Such engrafted mammals may carry canine immune cells that have either been generated in the mammal (e.g., following CD34+ canine hematopoietic stem cell injections) or generated in a canine donor and injected into the mammal (PBMCs). CD34 is a marker for stem/progenitor cells that are capable of producing every hematopoietic lineage. When injected in an immunocompromised mammal (e.g., a NSG mouse), they naturally migrate to the bone marrow and differentiate into the mature cell types of the immune system, along the established progenitor pathways. CD34+ cells can be readily isolated from mobilized peripheral blood, umbilical cord blood, or bone marrow. PBMCs ("peripheral blood mononuclear cells") include mature lymphocytes (B, T, and NK cells), monocytes, and macrophages. When injected in the immunocompromised mammal (e.g., a NSG mouse), PBMCs either remain in circulation (e.g., T cells), or die/migrate to other tissues (e.g., other cell types).

The parasitic worm used in the present invention is a filarial worm or a gastrointestinal parasite which is *Dirofilaria immitis, Strongyloides stercoralis* or *Haemonchus contortus.* Infections with parasitic filarial worms cause disease conditions generically known as filariasis. Filarial parasites and their life cycle are described in more detail elsewhere herein. They are vectored by arthropods, mature and mate in specific host tissues, and produce microfilariae. The Filarioidea include several families, such as Aproctidae, Creagrocercidae, Drilonematidae, Filariidae, Homungellidae, Mesidionematidae, Onchocercidae, Scolecophilidae, Setariidae, and Ungellidae. Examples of genera within these families include *Wuchereria, Brugia, Onchocerca, Mansonella, Dirofilaria, Parafilaria, Stenofilaria,* and *Loa.* Examples of filarial parasites include the following: *Wuchereria bancrofti; Brugia* species (e.g., *Brugia malayi,* and *Brugia timori*); *Mansonella* species (e.g., *Mansonella ozzardi, Mansonella perstans, Mansonella streptocerca,* and *Mansonella semiclarum* or *Mansonella perstans*)*; Dirofilaria immitis; Dirofilaria repens; Parafilaria* species (e.g., *Parafilaria bovicola* and *Parafilaria multipapillosa*); *Onchocerca* species (e.g., *Onchocerca volvulus, Onchocerca dermata, Onchocerca ochengi,* and *Onchocerca dukei*); *Stenofilaria assamensis*; and *Loa loa.* In one embodiment of the invention, the filarial parasite is *Dirofilaria immitis.*

In another example, the nematode parasite can be a gastrointestinal parasite. A gastrointestinal parasite refers to a parasite that infects the gastrointestinal tract. For example, the nematode can be from the Strongylida order or the Trichostrongyloidea superfamily (e.g., the *Haemonchus* genus). Trichostrongyloidea are divided into 14 families and 24 subfamilies. These worms have very small mouths and are found in a large number of hosts. They generally infect the stomach or intestine. In one embodiment of the invention, the parasite is *Haemonchus contortus* (barber's pole worm), which is described in more detail elsewhere herein. Adult worms attach to the abomasal mucosa and feed on blood. The disease caused by *Haemonchus* worms is called haemonchosis. In another example, the nematode parasite can be from the Rhabditida order (e.g., the Strongyloididae family or the *Strongyloides* genus). In one embodiment of the invention, the nematode parasite is *Strongyloides stercoralis* (threadworm), which is a human pathogenic parasitic roundworm causing the disease strongyloidiasis. The adult parasitic stage lives in tunnels in the mucosa of the small intestine. *S. stercoralis* has been reported in other mammals, including cats and dogs.

In a second aspect, the invention provides a method of determining the number of parasitic worms in one or more isolated tissue sample(s) of an immunocompromised rodent, preferably necropsied immunocompromised rodent, infected with a specific number of parasitic worm larvae and treated with a compound or combination of compounds to be tested, wherein the method comprises determining the number of parasitic worms, optionally adult parasitic worms, present in the isolated tissue sample(s); wherein the parasitic worm is a nematode and the parasitic worm is *Dirofilaria immitis, Strongyloides stercoralis* or *Haemonchus contortus* and wherein the rodent is an NOD scid IL2 receptor gamma chain knockout mouse (NSG) (NOD-SCID-IL2Ry -/-) or an NRG (NOD-Rag1^{null}IL2rg^{null}) immunodeficient mouse.

By infected tissue is meant a tissue in which that type of worm is found in an infected, but untreated immunocompromised rodent.

The worms counted in the tissue can be any stage of worm (e.g., microfilariae (Mf), L3, L4, L5, or adult worms). Likewise, eggs can be counted. In one embodiment, the number of adult worms is counted. Preferably, the tissue in which the worms are counted is a tissue in which that type of worm is typically found in an infected definitive host or a tissue in which that type of worm is found in an infected, but untreated immunocompromised rodent. For example, for *D. immitis,* the tissue can be muscle or skin. As another example, the tissue for a gastrointestinal parasite such as *Strongyloides stercoralis* or *Haemonchus contortus* can be the stomach, the small intestine, or another tissue from the gastrointestinal tract.

The worms can be counted at different time points post-infection. For example, the worms can be counted at about 1, 2, 3, 4, 5, 6, 7, 8, 9, 10, 11, 12, 13, 14, 15, or more weeks post-infection. In some embodiments, the worms are counted at about 2-10, 3-9, 4-8, 5-7, 6 weeks post-infection.

In some embodiments, the immunocompromised mouse used in the methods of the second aspect comprises engrafted immune cells. The engrafted immune cells can be from another species, such as engrafted immune cells from a definitive host of a particular parasitic worm. In some embodiments, the immunocompromised rodent may comprise engrafted hematopoietic stem cells. In some embodiments, the immunocompromised rodent may comprise engrafted mature immune cells. For example, the immunocompromised rodent can comprise engrafted canine immune cells. The engrafted immune cells can be CD34+ cells or hematopoietic stem cells (e.g., from umbilical cord blood or bone marrow). For example, about 10,000, 20,000, 30,000, 40,000, 50,000, 60,000, 70,000, 80,000, 90,000, 100,000, 10,000-100,000, 20,000-100,000, 30,000-100,000, 40,000-100,000, or 50,000-100,000 or more CD34+ cells can be injected into the immunocompromised rodent model. Alternatively, the engrafted immune cells can be mature immune cells, such as PBMCs ("peripheral blood mononuclear cells") including, for example, mature lymphocytes (B, T, and NK cells), monocytes, and macrophages. For example, about 1 × 10⁶, 2 × 10⁶, 3 × 10⁶, 4 × 10⁶, 5 × 10⁶, 6 × 10⁶, 7 × 10⁶, 8 × 10⁶, 9 × 10⁶, 10 × 10⁶, 15 × 10⁶, 20 × 10⁶, 5 × 10⁶ - 10 × 10⁶, 1 × 10⁶ - 20 × 10⁶, 2 × 10⁶ - 20 × 10⁶, 3 × 10⁶ - 20 × 10⁶, 4 × 10⁶ - 20 × 10⁶, or 5 × 10⁶ - 20 × 10⁶ or more PBMCs can be injected into the immunocompromised rodent.

The immunocompromised mouse can be infected in the methods of the invention by any suitable means. Examples of suitable means include subcutaneous injection, intraperitoneal injection, and oral administration. For example, for filarial worms such as *D. immitis,* the infection can be through administration of the larvae by subcutaneous injection or intraperitoneal injection. For gastrointestinal parasites, the infection can be, for example, through administration of the larvae by oral gavage.

The number of parasitic worm larvae used to infect the immunocompromised rodent in the methods of the second or third aspects can be any number sufficient to establish a complete infection. For example, about 5, 10, 15, 20, 25, 30, 35, 40, 45, 50, 60, 70, 80, 90, 100, 110, 120, 130, 140, 150, 200, 300, 400, 500, 1000, 2000, 3000, 4000, 5000, 25-5000, 6000, 7000, 8000, 9000, 10000, 1000-10000, 2000-9000, 3000-8000, 4000-7000, 5000-6000, 1000-5000, 5000-10000, 25-4000, 25-3000, 25-2000, 25-1000, 100-5000, 200-4000, 300-3000, 400-2000, 500-1000, 25-500, 50-200, or 50-100 parasitic worm larvae can be used. In one embodiment, about 50-100 parasitic worm larvae (e.g., *D. immitis* larvae) are used. In one embodiment, 500-1000 parasitic worm larvae (e.g., *Haemonchus contortus* larvae) are used. In one embodiment, 1000-5000 parasitic worm larvae (e.g., *Strongyloides stercoralis*) are used. The parasitic worm larvae can be any stage, such as any stage sufficient to infect the rodent. For example, the stage of parasitic worm (e.g., nematode or filarial worm) used can be microfilariae, L3 larvae, L4 larvae, L5 larvae, or young adult worms. In one embodiment, L3 larvae are used.

In some embodiments, the method can further comprise infecting a control immunocompromised rodent with a specific number of parasitic worm larvae, necropsying the control immunocompromised rodent and determining the number of worms present in a rodent tissue, and comparing the number of worms in the rodent tissue from the control (i.e., untreated) immunocompromised rodent with the number of worms in the rodent tissue from the treated immunocompromised rodent. Preferably, the control and the treated rodents are infected with the same number of parasitic worm larvae by the same means of infection. Preferably, the tissue samples from the control and the treated rodents are the same type of tissue isolated at the same time point post-infection.

In some embodiments, the method can further comprise comparing the parasiticidal efficacy of the test compound(s) against the parasiticidal efficacy of a compound known to have parasiticidal activity against the parasitic worm being tested. For example, the method can further comprise comparing the parasiticidal efficacy of the test compound(s) against the parasiticidal efficacy of either or both of a macrocyclic lactone (ML) parasiticidal compound or a depsipeptide parasiticidal compound. Examples of ML parasiticidal compounds include avermectin, ivermectin, doramectin, abamectin, milbemycin, and moxidectin. Examples of depsipeptide parasiticidal compound include emodepside.

In some examples, the efficacy of putative parasiticidal compounds may be tested against a target parasite up to about 95% faster than efficacy studies in dogs or other definitive host animals. In some examples, the efficacy of putative filaricidal and/or microfilaricidal compounds may be tested against a target parasite up to about 95% faster than efficacy studies in dogs. In some embodiments, emodepside and ivermectin eliminate HW parasites when said active pharmaceutical ingredients (APIs) were delivered orally to HW-infected NSG mice. To Applicants' knowledge, the data disclosed herein are the first to demonstrate that any immunocompromised rodent is capable of harboring, and promoting the development of, HW.

As used herein, a "parasiticidal" agent is one that kills parasitic worms at any stage of development, unless otherwise specified. As used herein, a "filaricidal" agent is one that kills filarial worms at any stage of development, unless otherwise specified. Similarly, a "microfilaricidal" agent is one that kills microfilariae. In some cases, an agent may be described as being "selective" for one or more stage(s) of parasitic worm or nematode or filarial worm development. In such cases, "selective" means that the agent exhibits greater efficacy against one developmental stage as compared with its efficacy against another developmental stage. In particular embodiments, selective agents are at least about 3, 10, 30, 100, 300, or 1000-fold more effective at killing one stage of parasitic worm or nematode or filarial worm versus another stage. In some embodiments, selective agents exhibit no detectable efficacy against non-target developmental stages.

In an example, microfilariae are administered to the immunocompromised rodents, to allow for the screening and evaluation of APIs that selectively target this earlier stage of the filarial parasites. Adult filarial nematodes live in a tissue or the circulatory system of vertebrates (the "definitive hosts"). These mature worms release microfilariae into the bloodstream of the vertebrate host. The microfilariae are then taken up by blood-feeding arthropod vectors (the "intermediate hosts"). In the intermediate host, the microfilariae develop into infective larvae (e.g., L3 *D. immitis* larvae), which may be transmitted to a new definitive host. The presence of microfilariae in the host bloodstream is referred to as "microfilaraemia." Although the methods of the example specify that microfilariae are administered rather than other stages of filarial worm, the type of immunocompromised rodents used, the type of filarial worms used, the means of infection, the number of microfilariae used to infect the immunocompromised rodent, the stage of worms counted in infected tissue, the use of control immunocompromised rodents, the comparison of parasiticidal efficacy (filarial efficacy) to other compounds, and any other features of the methods can all be as described for the third aspect.

In other embodiments of any of the disclosed methods, any larval stage may be studied and/or targeted, including, but not limited to, Mf, L3, L4, L5 and young adult. In one embodiment, the L3 larval stage is used.

In a third aspect, the invention provides one or more filaricidal compound(s) identified by the method of any one of claims 1 to 9, selected from the group consisting of 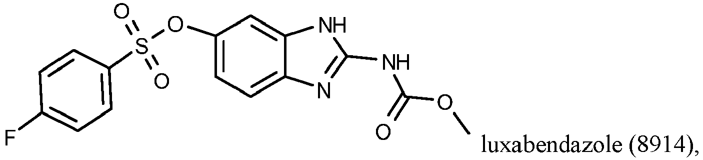 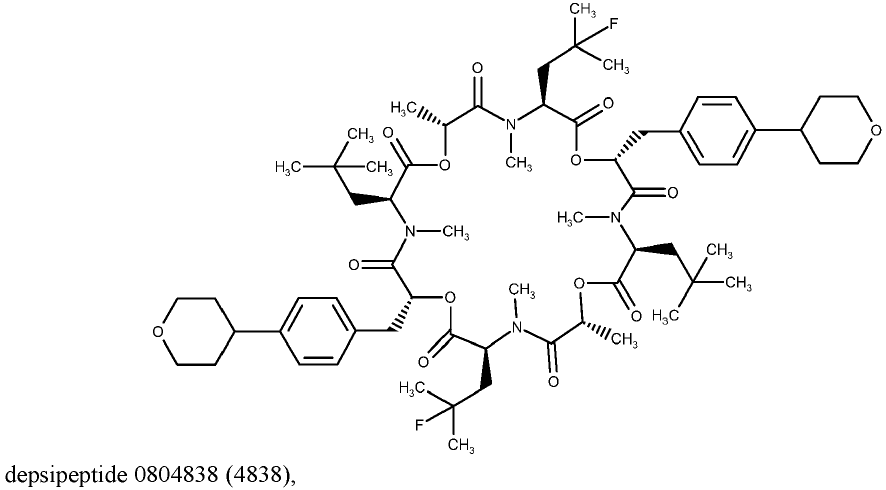 and 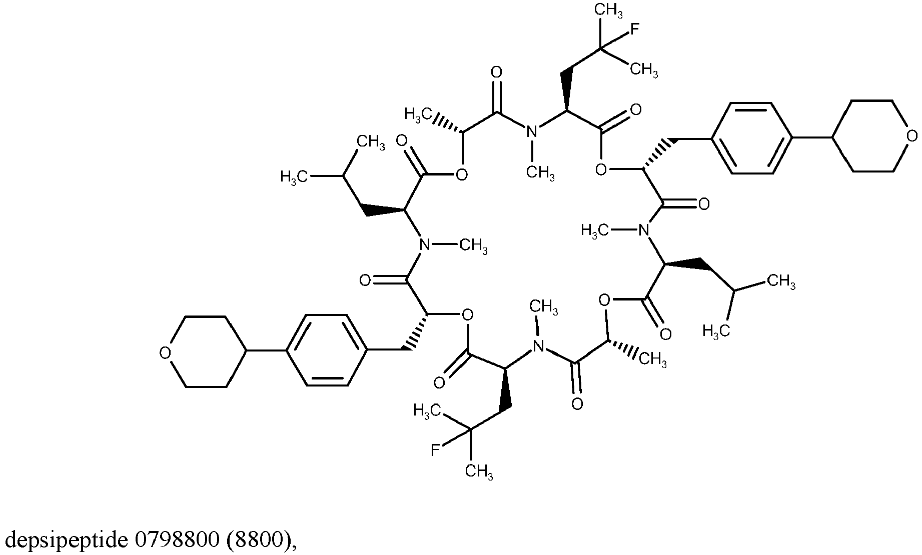 for use in a method of treating filariasis in a subject in need thereof, wherein the filariasis is dirofilariasis.

The subject can be, for example, a human, a non-human animal, a non-human mammal, a dog, a sheep, a goat, or a cow. In one example, the parasitic worm is a nematode. The filarial worm can be *D. immitis,* and the subject can be a dog. In another example, the nematode can be a gastrointestinal parasite. As another example, the nematode can be *Strongyloides stercoralis* (threadworm), and the subject can be a human or a dog. As another example, the nematode can be *Haemonchus contortus,* and the subject can be a ruminant. A ruminant is a mammal that chews the cud regurgitated from its rumen, such as a sheep, a goat, or a cow.

### Definitions

The term "about," as used herein, means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. In one aspect, the term "about" means plus or minus 20% of the numerical value of the number with which it is being used. Therefore, about 50% means in the range of 45%-55%. Numerical ranges recited herein by endpoints include all numbers and fractions subsumed within that range (e.g., 1 to 5 includes 1, 1.5, 2, 2.75, 3, 3.90, 4, and 5). It is also to be understood that all numbers and fractions thereof are presumed to be modified by the term "about."

By "animal" it is intended mammals, birds, and the like. Animal or host includes mammals and human. The animal may be selected from the group consisting of, but not limited to: equine, canine, feline, ovine, bovine, swine, caprine, avian, primate, and fish. The term "animal" also includes an individual animal in all stages of development, including embryonic and fetal stages.

Unless otherwise explained, all technical and scientific terms used herein have the same meaning as commonly understood by one of ordinary skill in the art to which this disclosure belongs. The singular terms "a", "an", and "the" include plural referents unless context clearly indicates otherwise. Similarly, the word "or" is intended to include "and" unless the context clearly indicate otherwise.

The term "filariasis" as used herein refers to helminth infections that are caused by filarial nematodes. An infection is the colonization of a host organism by parasite species. Infections with human filarial nematodes can cause lymphatic filariasis or onchocerciasis. The term "lymphatic filariasis" refers to an infection with the nematodes *Wuchereria bancrofti, Brugia malayi* or *Brugia timori.* The term "onchocerciasis" refers to an infection with the nematode *Onchocerca volvulus.* Lymphatic filariasis may cause hydrocoele, lymphoedema, and elephantiasis. Onchocerciasis may cause skin inflammation and blindness, so called River Blindness. In dogs, an infection with nematode species called *Dirofilaria immitis* (*D. immitis*) and *Dirofilaria repens* (*D. repens*) causes dirofilariasis. The former nematode species cause HW disease, while the latter causes subcutaneous dirofilariasis. As such, when the term "heartworm" (HW) is used to refer to the nematode, it is referring to *D. immitis,* not *D*. *repens.*

As used herein, the term "prophylactic treatment" refers to either preventing or inhibiting the development of a clinical condition or disorder or delaying the onset of a pre-clinically evident stage of a clinical condition or disorder. The term "prophylactic treatment" according to the present invention is to be understood as meaning that the compositions according to the present invention can be applied before symptoms of the infection are manifest. The term "prophylactic treatment" is to be understood as meaning a medical treatment. The compounds identified using the immunocompromised rodent model, according to the present invention can, for example, be used in a prophylactic treatment.

The term "therapeutically effective amount" is used herein to mean an amount or dose sufficient to cause an improvement in a clinically significant condition in the subject.

As used herein, "IVM" is ivermectin; "EMO" is emodepside; "8800" is depsipeptide 0798800; "4838" is depsipeptide 0804838; and "8914" is luxabendazole; and "6557" is a DI3 analog. "3615," "8800," "4838," "7823," "5010," and "2677" are depsipeptide analogs described in US 2017/0022253 A1 and US 2017/0233438 A1. Structures for these compounds are provided below.

Unless otherwise defined, the technical and scientific terms used herein have the same meaning as commonly understood by one of ordinary skill in the art to which the present invention belongs.

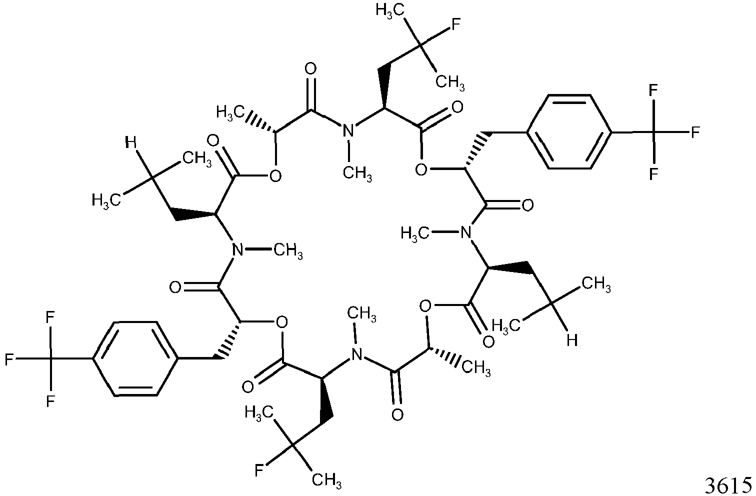

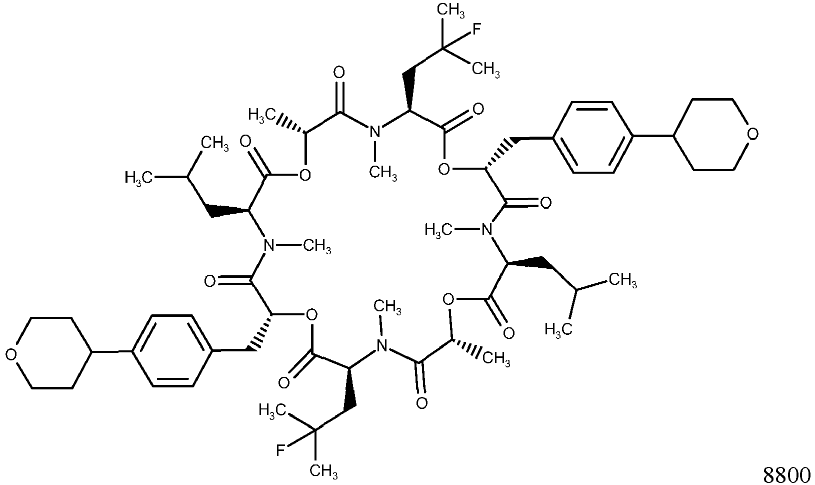

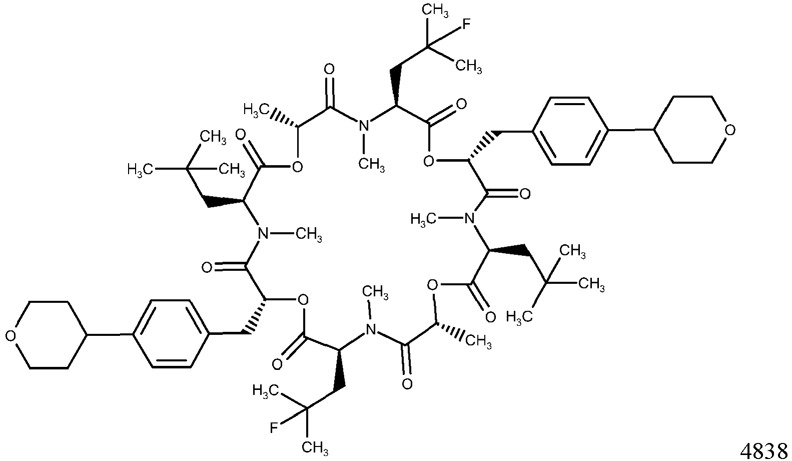

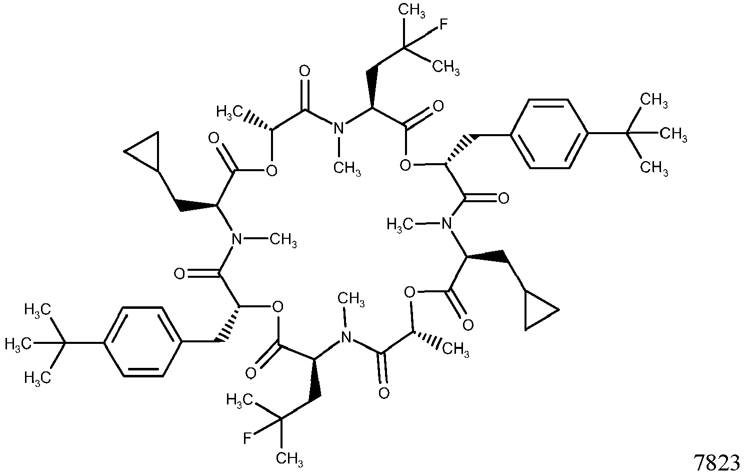

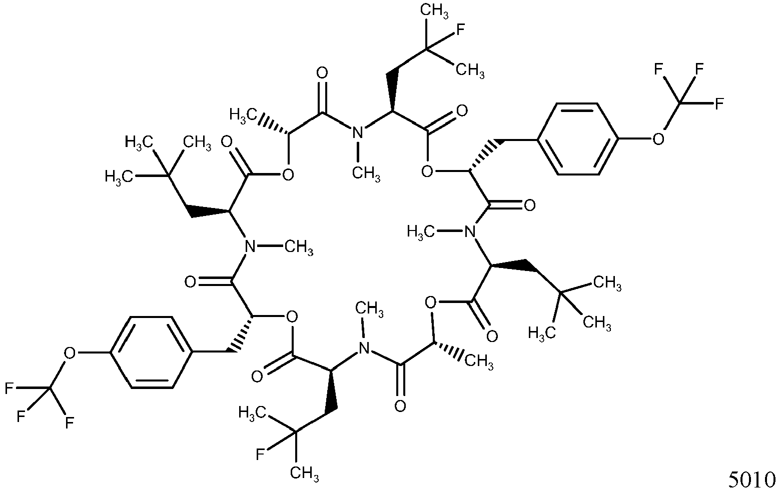

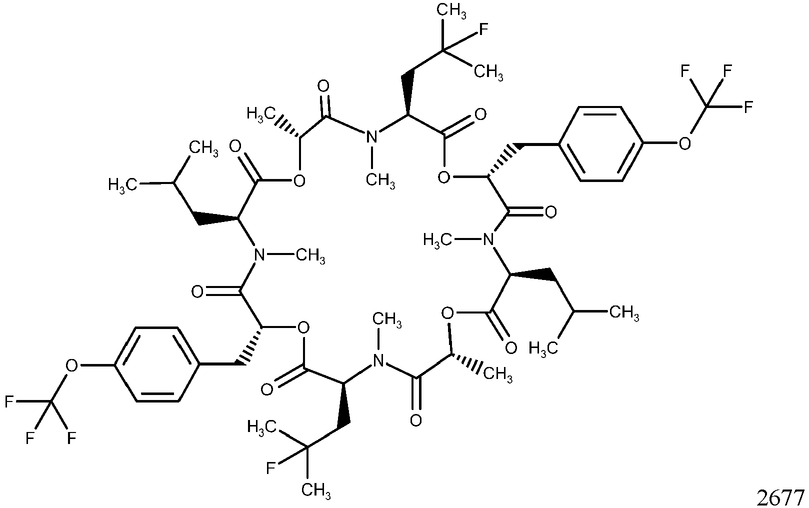

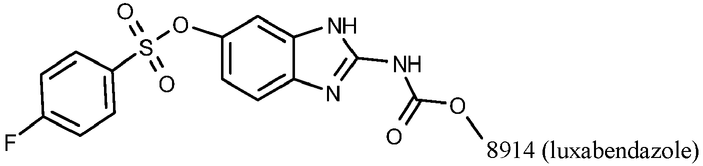

In a fourth aspect, the invention provides an immunocompromised rodent infected with one or more adult parasitic worms, wherein the parasitic worm is a nematode and wherein the parasitic worm is *Dirofilaria immitis, Strongyloides stercoralis* or *Haemonchus contortus* and the rodent is an NOD scid IL2 receptor gamma chain knockout mouse (NSG) (NOD-SCID-IL2Ry -/-) or an NRG (NOD-Rag1^{null}IL2rg^{null}) immunodeficient mouse. In a fifth aspect, provided is the use of an immunocompromised rodent of the fourth aspect above for modeling complete infection of a parasitic worm outside of the worm's definitive host or for characterizing changes in sensitivity to a parasiticidal or filaricidal agent during parasitic or filarial worm infection.

In a sixth aspect, the invention provides the use of an immunocompromised rodent for modeling complete infection of a parasitic worm outside of the worm's definitive host, wherein the rodent is an NOD scid IL2 receptor gamma chain knockout mouse (NSG) (NOD-SCID-IL2Ry -/-) or an NRG (NOD-Rag1^{null}IL2rg^{null}) immunodeficient mouse and the parasitic worm is a nematode and the parasitic worm is *Dirofilaria immitis, Strongyloides stercoralis* or *Haemonchus contortus.*

The following examples are simply intended to further illustrate and explain the present invention.

### EXAMPLES

For the following studies, *D. immitis* were obtained from FR3, the Merial insectary and Joe Prullage's group at MRC. As used herein, "FR3" refers the Filariasis Research Reagent Resource Center at the University of Georgia Vet School, which supplied Applicants with ML-susceptible 2005 Missouri strain *Di.* In general, worms were recovered from mosquitos and shipped overnight in media and antibiotics. Worms were washed and counted for use in mice.

Mice were injected subcutaneously or via intraperitoneal injection. For the recovery of parasites, mice were exsanguinated for serum collection, skinned and the fascia scored, the viscera (pluck) removed, and the muscle fascia scored. All pieces were left to soak overnight room temperature in RPMI media supplemented with 10% FBS and Pen/strep antibiotics. The following morning, approximately 18 hours later, worms were counted and fixed in 95% ethanol/5% glycerol. Worms were mounted in glycerine jelly and then measured using cellSens software.

### Example 7 - Development and Validation of an Intermediate in Vivo Model of Canine Heartworm Infection

Canine heartworm (HW), *D. immitis,* related to the human river-blindness parasite *O*. *volvulus,* is transmitted via a mosquito vector that delivers infective L3 larva to hosts. The parasite matures through several larval stages, migrating through host tissue to the pulmonary arteries and heart. Adult heartworms can grow to 20-30 cm in length. Adults produce circulating microfilariae that are transferred to the mosquito in a bloodmeal. Once internalized, the microfilaria mature to infective L3 larvae, completing the cycle.

However, prior to the present disclosure, there existed a significant challenge for translational biologists: *Dirofilaria immitis* (*Di*) larvae fail to develop in immunocompetent rodents (see Table 2). "Mouse #" refers to the identifier for a particular mouse. The numbers in the "Pluck," "Upper Muscles," "Lower Muscles," "Skin," "Total," and "Average Recovery" columns refer to number of *Di* recovered. Timelines, cost, and risk were thus increased due to sole reliance upon canine studies.

**Table 2. D. immitis larvae do not survive in C57BL6 mice (comparative).**

| **C57/BL6 Mouse #** | **Pluck** | **Upper Muscles** | **Lower Muscles** | **Skin** | **Total** | **Average Recovery** |
|---|---|---|---|---|---|---|
| 1 | 0 | 0 | 0 | 0 | 0 | 0 |
| 2 | 0 | 0 | 0 | 0 | 0 | |
| 3 | 0 | 0 | 0 | 0 | 0 | |
| 4 | 0 | 0 | 0 | 0 | 0 | |

Surprisingly, Applicants found that immunodeficient NSG mice were susceptible to *D. immitis* infection and complete development. Following subcutaneous (SQ) infection with 100 infective L3 stage larva, *D. immitis* can be recovered from NSG mouse tissues (Table 3), indicating migration through the murine host similar to that which occurs in canine infections.

**Table 3. Recovery of D. immitis from NSG mice 6 weeks post SQ infection.**

| **NSG Mouse #** | **Pluck** | **Upper Muscles** | **Lower Muscles** | **Skin** | **Total** | **Average Recovery** |
|---|---|---|---|---|---|---|
| 1 | 0 | 10 | 10 | 13 | 33 | 25 |
| 2 | 0 | 9 | 1 | 5 | 15 | |
| 3 | 0 | 3 | 11 | 11 | 25 | |
| 4 | 0 | 12 | 8 | 8 | 28 | |

As disclosed herein, the mouse model of canine HW infection promises to accelerate discovery of new pharmaceutical treatments by reducing timelines (6 weeks in mouse, compared to 6 months in canines) and costs.

Similar results were obtained when *D. immitis* L3 larvae were introduced into NSG or C57BL6 mice via the intraperitoneal (IP) route (Table 4).

**Table 4. Recovery of D. immitis from NSG mice 6 weeks post IP infection.**

| **Source** | **Sex** | **Mouse #** | **Pluck** | **Upper Muscles** | **Lower Muscles** | **Skin** | **Total** | **Average Recovery** |
|---|---|---|---|---|---|---|---|---|
| NSG | M | 1 | 0 | 5 | 1 | 2 | 8 | 8 |
| | M | 2 | 0 | 3 | 3 | 2 | 8 | |
| C57BL6 | M | 3 | 0 | 0 | 0 | 0 | 0 | 0 |
| | M | 4 | 0 | 0 | 0 | 0 | 0 | |
| | M | 5 | 0 | 0 | 0 | 0 | 0 | |

To determine the effect of varying the dose, NSG mice were injected SQ as described above, this time with 25, 50 or 100 *D. immitis* L3 larvae (Table 5). "Mouse #" refers to the identifier for a particular mouse. The numbers in the "Pluck," "Upper Muscles," "Lower Muscles," "Skin," "Total," and "Average Recovery" columns refer to number of *Di* recovered. "% Recovery" refers to percent of the original 25, 50, or 100 *Di* recovered from the mouse. And while there was a statistically significant dose-dependent increase in average recovery, percent recovery did not change significantly across the tested doses.

**Table 5. Recovery of D. immitis from NSG mice 6 weeks post SQ infection with 25, 50, or 100 L3 larvae.**

| **Dose** | **Sex** | **Mouse #** | **Pluck** | **Upper Muscles** | **Lower Muscles** | **Skin** | **Total** | **Average Recovery** | **% Recovery** |
|---|---|---|---|---|---|---|---|---|---|
| 25 | F | 9 | 0 | 1 | 0 | 4 | 5 | 4 | 16 |
| | M | 11 | 0 | 2 | 0 | 0 | 2 | | |
| | M | 12 | 0 | 5 | 0 | 2 | 7 | | |
| | F | 16 | 1 | 0 | 0 | 2 | 3 | | |
| | F | 17 | 0 | 0 | 1 | 2 | 3 | | |
| | F | 18 | 0 | 3 | 1 | 0 | 4 | | |
| 50 | F | 7 | 0 | 4 | 1 | 2 | 7 | 11 | 22 |
| | F | 8 | 1 | 2 | 7 | 4 | 14 | | |
| | F | 10 | 0 | 1 | 4 | 5 | 10 | | |
| | M | 13 | 0 | 6 | 0 | 3 | 9 | | |
| | M | 14 | 0 | 6 | 6 | 4 | 16 | | |
| | M | 15 | 0 | 2 | 2 | 7 | 11 | | |
| 100 | M | 1 | 0 | 2 | 5 | 10 | 17 | 21 | **21** |
| | M | 2 | 0 | 6 | 8 | 8 | 22 | | |
| | M | 3 | 0 | 3 | 10 | 9 | 22 | | |
| | M | 4 | 1 | 2 | 4 | 1 | 8 | | |
| | F | 5 | 1 | 14 | 6 | 11 | 32 | | |
| | F | 6 | 0 | 11 | 8 | 5 | 24 | | |
| ** ANOVA indicates each group is different from each other based on actual recovery numbers | | | | | | | | | |
| ***Same analysis on % survival shows no difference between groups | | | | | | | | | |

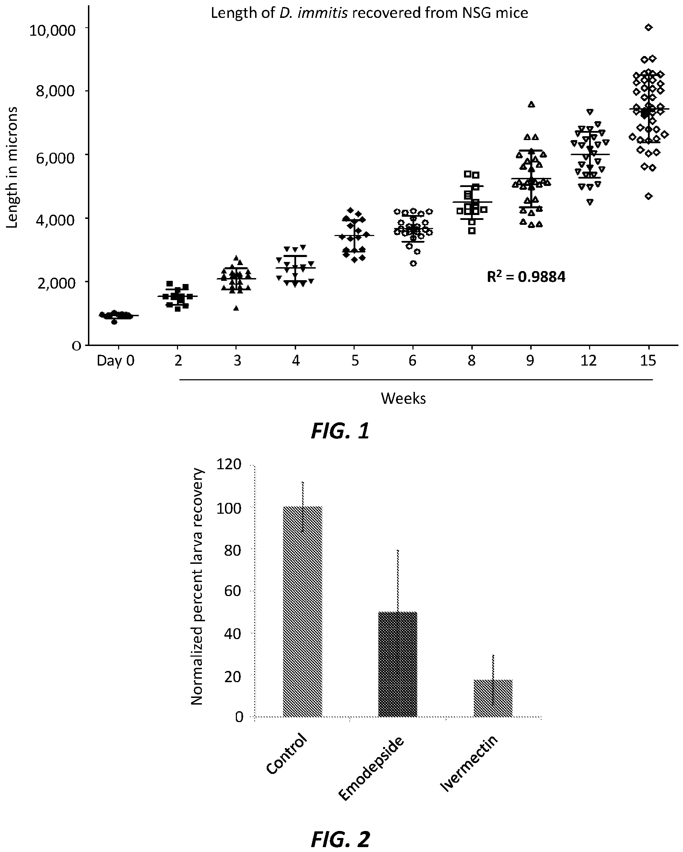

To test the linearity of the NSG mouse model of *D. immitis* infection, NSG mice were next infected with 100 L3 per mouse, using methods described above. Recovery and measurements were performed as above, and lengths were reported as a function of time post-infection (FIG. 1). As indicated, the model is significantly and surprisingly linear, with a calculated R² value of 0.9884.

To test whether HW could be eliminated from the NSG mouse model by pharmaceutical compounds, Applicants tested known filaricidal compounds, emodepside (EMO) and ivermectin (IVM). NSG mice were dosed orally with 1 mg/kg EMO or 3 mg/kg IVM, at Days 1, 15, and 30 post-infection. As indicated in FIG. 2, both compounds significantly reduced HW parasite load. In view of these promising data, Applicants envision that the immunocompromised rodent model may be successfully applied to a wide range of filarial parasites to predict compound efficacy in animals, including canines. Applicants further envision that HW parasites in NSG mice may be physiologically similar to those from their normal, definitive canine hosts.

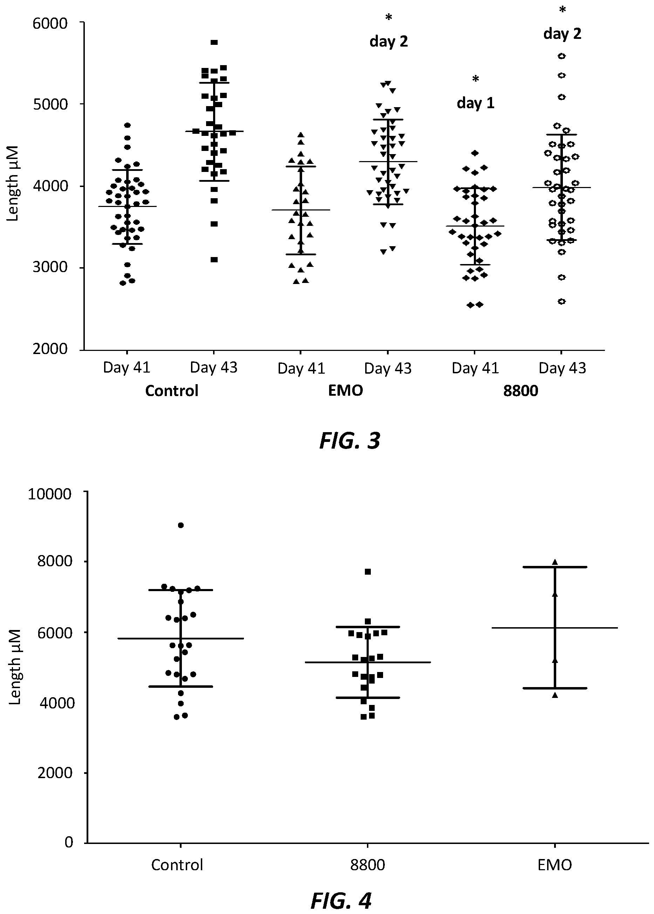

In another example, NSG mice were infected on Day 0 with 50 *Di* L3 larvae. The mice were then treated with 1mg/kg EMO or 8800 on Day 30, and recovery was conducted on Days 41 to 43. FIG. 3 shows the length of *Di* recovered from the mice at Days 41 and 43, and Table 6 provides survival data, recovery data, and summary statistics. "#" refers to the mouse identifier for a particular mouse. "Survival" refers to the number of *Di* recovered from the mouse. "% Rec" refers to percent of the original 50 *Di* recovered from the mouse. "% Red" refers to percent reduction in the percent of *Di* recovered compared to control.

**Table 6. Summary data for Di-infected NSG mice treated with Control, EMO, or 8800.**

| **Control** | | | **EMO** | | | **8800** | | |
|---|---|---|---|---|---|---|---|---|
| # | Survival | % Rec | # | Survival | % Rec | # | Survival | % Rec |
| 1 | 18 | 36 | 13 | 15 | 30 | 29 | 14 | 28 |
| 3 | 18 | 36 | 14 | 13 | 26 | 30 | 26 | 52 |
| 5 | 21 | 42 | 17 | 12 | 24 | 33 | 12 | 24 |
| 9 | 20 | 40 | 18 | 15 | 30 | 34 | 18 | 36 |
| 10 | 23 | 46 | 19 | 16 | 32 | 35 | 12 | 24 |
| | | | 20 | 14 | 28 | 36 | 12 | 24 |
| Mean | 20 | 40 | Mean | 14 | 28 | Mean | 16 | 31 |
| SD | 2 | 4 | SD | 1 | 3 | SD | 6 | 11 |
| | | | % Red | 30% | | % Red | 23% | |
| | | | P value | 0.019 | | P value | 0.069 | |

In another example, NSG mice were infected at Day 0 with 50 *Di* L3 larvae. The mice were then treated with Control; 1 mg/kg EMO; or 8800 on Days 30 and 60. FIG. 4 shows the lengths of *Di* recovered at Days 83 and 84, and Table 7 presents the survival data, recovery data, and summary statistics.

**Table 7. Summary data for Di-infected NSG mice treated with Control, EMO, or 8800.**

| **Control** | | | **EMO** | | | **8800** | | |
|---|---|---|---|---|---|---|---|---|
| # | Survival | % Rec | # | Survival | % Rec | # | Survival | % Rec |
| 6 | 11 | 22 | 15 | 1 | 2 | 31 | 6 | 12 |
| 7 | 5 | 10 | 16 | 0 | 0 | 32 | 3 | 6 |
| 8 | 7 | 14 | 21 | 1 | 2 | 37 | 7 | 14 |
| 11 | 5 | 10 | 23 | 0 | 0 | 38 | 2 | 4 |
| 12 | 6 | 12 | 25 | 3 | 6 | 41 | 2 | 4 |
| | | | 26 | 1 | 2 | 42 | 5 | 10 |
| | | | 27 | 0 | 0 | | | |
| | | | 28 | 3 | 6 | | | |
| Mean | 7 | 14 | Mean | 1 | 2 | Mean | 4 | 8 |
| SD | 2 | 5 | SD | 1 | 2 | SD | 2 | 4 |
| | | | % Red | 86% | | % Red | 43% | |
| | | | P value | 0.000 | | P value | 0.021 | |

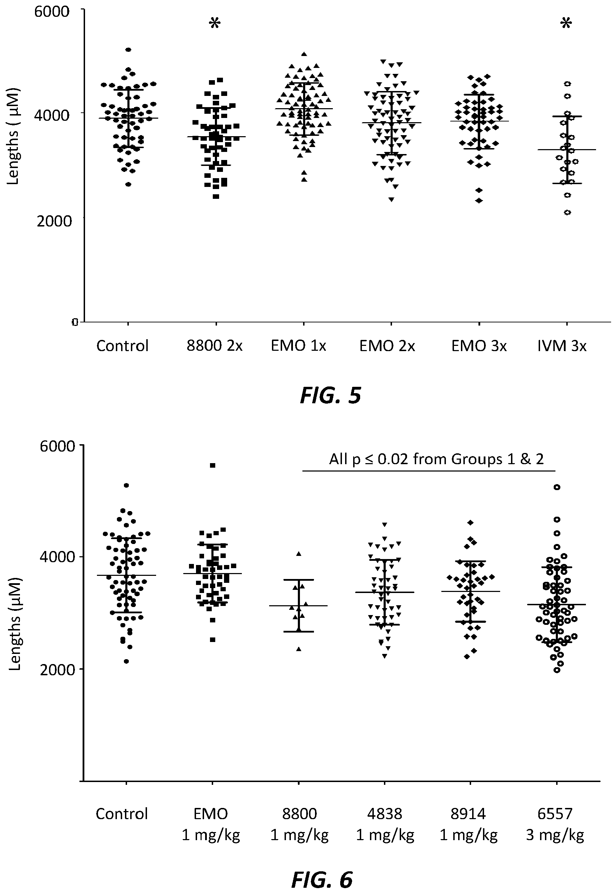

In another example, NSG mice were infected with 50 *Di* L3 per mouse, then treated according to the following: Control; 2X 8800 (1 mg/kg) at Days 1 and 30; 1X EMO (1 mg/kg) at Day 30; 2X EMO (1 mg/kg) at Days 1 and 30; 3X EMO (1 mg/kg) at Days 1, 15, and 30; or 3X IVM (3 mg/kg) at Days 1, 15, and 30. FIG. 5 is a graph showing the length of *Di* recovered at Days 41 to 43, and Table 8 presents the survival data, recovery data, and summary statistics.

**Table 8. Summary data for Di-infected NSG mice treated with Control, 8800, EMO, or IVM.**

| **Control** | | | **2x 8800** | | | **1x EMO** | | | **2x EMO** | | | **3x EMO** | | | **3x IVM** | | |
|---|---|---|---|---|---|---|---|---|---|---|---|---|---|---|---|---|---|
| # | Survival | % Rec | # | Survival | % Rec | # | Survival | % Rec | # | Survival | % Rec | # | Survival | % Rec | # | Survival | % Rec |
| 1 | 16 | 32 | 7 | Died 1/14/16 | | 14 | 21 | 42 | 21 | 13 | 26 | 28 | 14 | 28 | 35 | 6 | 12 |
| 2 | 16 | 32 | 8 | 16 | 32 | 15 | 22 | 44 | 22 | 11 | 22 | 29 | 10 | 20 | 36 | 5 | 10 |
| 3 | 16 | 32 | 9 | 11 | 22 | 16 | 18 | 36 | 23 | 14 | 28 | 30 | 14 | 28 | 37 | 1 | 2 |
| 4 | 15 | 30 | 10 | 8 | 16 | 17 | 14 | 28 | 24 | 13 | 26 | 31 | 7 | 14 | 38 | 3 | 6 |
| 5 | 16 | 32 | 11 | 6 | 12 | 18 | 14 | 28 | 25 | 13 | 26 | 32 | 11 | 22 | 39 | 4 | 8 |
| 6 | 22 | 44 | 12 | 13 | 26 | 19 | 23 | 46 | 26 | 15 | 30 | 33 | 2 | 4 | 40 | 2 | 4 |
| | | | 13 | 10 | 20 | 20 | 23 | 46 | 27 | 21 | 42 | 34 | 3 | 6 | 41 | 1 | 2 |
| Mean | 17 | 34 | Mean | 11 | 21 | Mean | 19 | 39 | Mean | 14 | 29 | Mean | 9 | 17 | Mean | 3 | 6 |
| SD | 3 | 5 | SD | 4 | 7 | SD | 4 | 8 | SD | 3 | 6 | SD | 5 | 10 | SD | 2 | 4 |
| | | | % Red | 38% | | % Red | 0% | | % Red | 15% | | % Red | 50% | | % Red | 82% | |
| | | | P value | 0.005 | | P value | 0.218 | | P value | 0.201 | | P value | 0.000 | | P value | 0.000 | |

In another example, NSG mice were infected with 50 *Di* L3 per mouse, then treated according to the following: EMO (1 mg/kg); 8800 (1 mg/kg); 4838 (1 mg/kg); 8914 (1 mg/kg); and 6557 (3 mg/kg). FIG. 6 is a graph showing the lengths of *Di* recovered at Days 41 to 43; and, Table 9 presents the survival data, recovery data, and summary statistics.

**Table 9. Summary data for Di-infected NSG mice treated with Control, EMO, 8800, 4838, 8914, or 6557.**

| **Control** | | | **EMO** | | | **8800** | | | **4838** | | | **8914** | | | **6557** | | |
|---|---|---|---|---|---|---|---|---|---|---|---|---|---|---|---|---|---|
| # | Survival | % Rec | # | Survival | % Rec | # | Survival | % Rec | # | Survival | % Rec | # | Survival | % Rec | # | Survival | % Rec |
| 1 | 12 | 24 | 8 | 10 | 20 | 14 | 8 | 16 | 20 | 7 | 14 | 26 | 16 | 32 | 32 | 21 | 42 |
| 2 | 13 | 26 | 9 | 8 | 16 | 15 | 0 | 0 | 21 | 16 | 32 | 27 | 14 | 28 | 33 | 13 | 26 |
| 3 | 19 | 38 | 10 | 11 | 22 | 16 | 4 | 8 | 22 | 15 | 30 | 41 | 24 | 48 | 34 | 13 | 26 |
| 4 | 17 | 34 | 11 | 13 | 26 | 17 | 4 | 8 | 23 | 13 | 26 | 29 | 12 | 24 | 35 | Died 3/18 | |
| 5 | 19 | 38 | 12 | 12 | 24 | 18 | 5 | 10 | 24 | 15 | 30 | 30 | Died 3/14 | | 36 | 19 | 38 |
| 6 | 20 | 40 | 13 | 12 | 24 | 19 | 4 | 8 | 25 | 6 | 12 | 31 | Died 2/19 | | 37 | 27 | 54 |
| 7 | 14 | 28 | | | | 39 | 8 | 16 | 40 | 14 | 28 | 38 | Died 3/14 | | 42 | Died 3/3 | |
| | | | | | | | | | | | | 28 | Died 2/19 | | | | |
| Mean | 16 | 33 | Mean | 11 | 22 | Mean | 5 | 9 | Mean | 12 | 25 | Mean | 17 | 33 | Mean | 19 | 37 |
| SD | 3 | 7 | SD | 2 | 4 | SD | 3 | 6 | SD | 4 | 8 | SD | 5 | 11 | SD | 6 | 12 |
| | | | % Red | 33% | | % Red | 73% | | % Red | 24% | | % Red | 0% | | % Red | 0% | |
| | | | P value | 0.020 | | P value | 0.000 | | P value | 0.062 | | P value | 0.930 | | P value | 0.314 | |

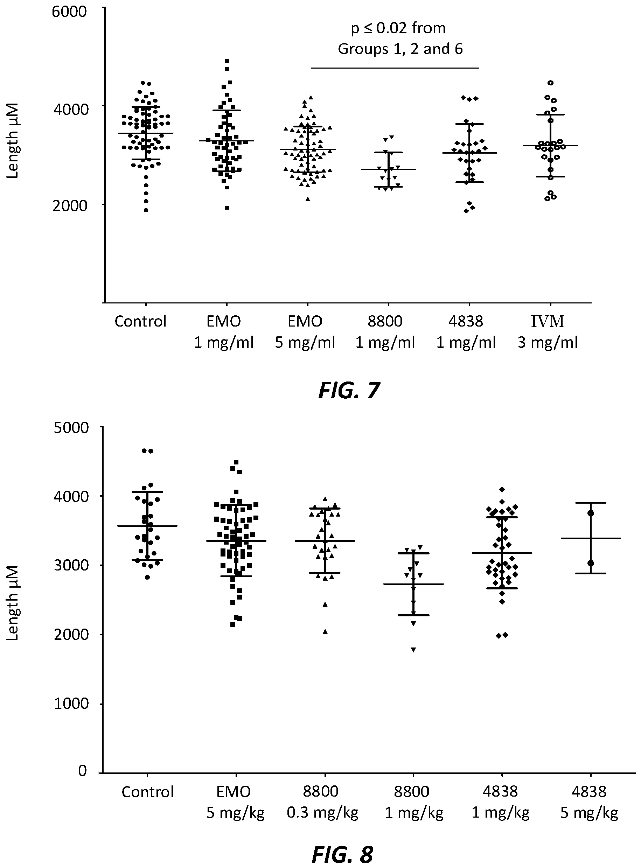

In another example, NSG mice were infected with 50 *Di* L3 Merial worms (not FR3 worms) per mouse, then treated on Days 1, 15, and 30 according to the following: EMO (1 mg/kg); EMO (5 mg/kg); 8800 (1 mg/kg); 4838 (1 mg/kg); or IVM (3 mg/kg). FIG. 7 is a graph showing the lengths of *Di* recovered at Days 41 to 43, and Table 10 presents the survival data, recovery data, and summary statistics.

**Table 10. Summary data for Di-infected NSG mice treated with Control, EMO low dose, EMO high dose, 8800, 4838, or IVM.**

| **Control** | | | **EMO low dose** | | | **EMO High dose** | | | **8800** | | | **4838** | | | **IVM** | | |
|---|---|---|---|---|---|---|---|---|---|---|---|---|---|---|---|---|---|
| # | Survival | % Rec | # | Survival | % Rec | # | Survival | % Rec | # | Survival | % Rec | # | Survival | % Rec | # | Survival | % Rec |
| 1 | 17 | 34 | 7 | 12 | 24 | 15 | 18 | 36 | 23 | 8 | 16 | 31 | 8 | 16 | 39 | 16 | 32 |
| 2 | 14 | 28 | 8 | 16 | 32 | 16 | 24 | 48 | 24 | 0 | 0 | 32 | 18 | 36 | 40 | 2 | 4 |
| 3 | 17 | 34 | 9 | 11 | 22 | 17 | 24 | 48 | 25 | 6 | 12 | 33 | 10 | 20 | 41 | 6 | 12 |
| 4 | 17 | 34 | 10 | 7 | 14 | 18 | 26 | 52 | 26 | 0 | 0 | 34 | 10 | 20 | 42 | 8 | 16 |
| 5 | 25 | 50 | 11 | 13 | 26 | 19 | 34 | 68 | 27 | 6 | 12 | 35 | 6 | 12 | 43 | 6 | 12 |
| 6 | 18 | 36 | 12 | 12 | 24 | 20 | 24 | 48 | 28 | 4 | 8 | 36 | 12 | 24 | 44 | 8 | 16 |
| 47 | 11 | 22 | 13 | 8 | 16 | 21 | 28 | 56 | 29 | 6 | 12 | 37 | 10 | 20 | 45 | 12 | 24 |
| | | | 14 | 10 | 20 | 22 | 24 | 48 | 30 | 16 | 32 | 38 | 8 | 16 | 46 | 14 | 28 |
| Mean | 17 | 34 | Mean | 11 | 22 | Mean | 25 | 51 | Mean | 6 | 12 | Mean | 10 | 21 | Mean | 9 | 18 |
| SD | 4 | 9 | SD | 3 | 6 | SD | 5 | 9 | SD | 5 | 10 | SD | 4 | 7 | SD | 5 | 9 |
| | | | % Red | 35% | | % Red | 0% | | % Red | 65% | | % Red | 38% | | % Red | 47% | |
| | | | P value | 0.010 | | P value | 0.001 | | P value | 0.000 | | P value | 0.004 | | P value | 0.000 | |

In another example, NSG mice were infected with 50 *Di* L3 per mouse, then treated on Days 1, 15, and 30 according to the following: EMO (5 mg/kg); 8800 (0.3 mg/kg); 8800 (1 mg/kg); 4838 (1 mg/kg); or 4838 (5 mg/kg). FIG. 8 is a graph showing the lengths of *Di* recovered at Days 41 to 42, and Table 11 presents the survival data, recovery data, and summary statistics.

**Table 11. Summary data for Di-infected NSG mice treated with Control, EMO, 8800, or 4838.**

| **Control** | | | **EMO 5 mg/kg** | | | **8800 0.3 mg/kg** | | | **8800 1 mg/kg** | | | **4838 1 mg/kg** | | | **4838 5 mg/kg** | | |
|---|---|---|---|---|---|---|---|---|---|---|---|---|---|---|---|---|---|
| # | Survival | % Rec | # | Survival | % Rec | # | Survival | % Rec | # | Survival | % Rec | # | Survival | % Rec | # | Survival | % Rec |
| 1 | 13 | 26 | 6 | 11 | 22 | 11 | 8 | 16 | 16 | 4 | 8 | 21 | 15 | 30 | 26 | 2 | 4 |
| 2 | 9 | 18 | 7 | 17 | 34 | 12 | 18 | 36 | 17 | 2 | 4 | 22 | 14 | 28 | 27 | 1 | 2 |
| 3 | 14 | 28 | 8 | 7 | 14 | 13 | 18 | 36 | 18 | 3 | 6 | 23 | 9 | 18 | 28 | 0 | 0 |
| 4 | 13 | 26 | 9 | 11 | 22 | 14 | 8 | 16 | 19 | 2 | 4 | 24 | 10 | 20 | 29 | 2 | 4 |
| 5 | 11 | 22 | 10 | 14 | 28 | 15 | 18 | 36 | 20 | 1 | 2 | 25 | 6 | 12 | 30 | 1 | 2 |
| 31 | 13 | 26 | 32 | 18 | 36 | 33 | 7 | 14 | 34 | 8 | 16 | 35 | 4 | 8 | 36 | 1 | 2 |
| | | | | | | | | | | | | | | | | | |
| Mean | 12 | 24 | Mean | 13 | 26 | Mean | 13 | 26 | Mean | 3 | 7 | Mean | 10 | 19 | Mean | 1 | 2 |
| SD | 2 | 4 | SD | 4 | 8 | SD | 6 | 11 | SD | 3 | 5 | SD | 4 | 9 | SD | 1 | 2 |
| | | | % Red | 0% | | % Red | 0% | | % Red | 71% | | % Red | 21% | | % Red | 92% | |
| | | | P value | 0.692 | | P value | 0.751 | | P value | 0.000 | | P value | 0.240 | | P value | 0.000 | |

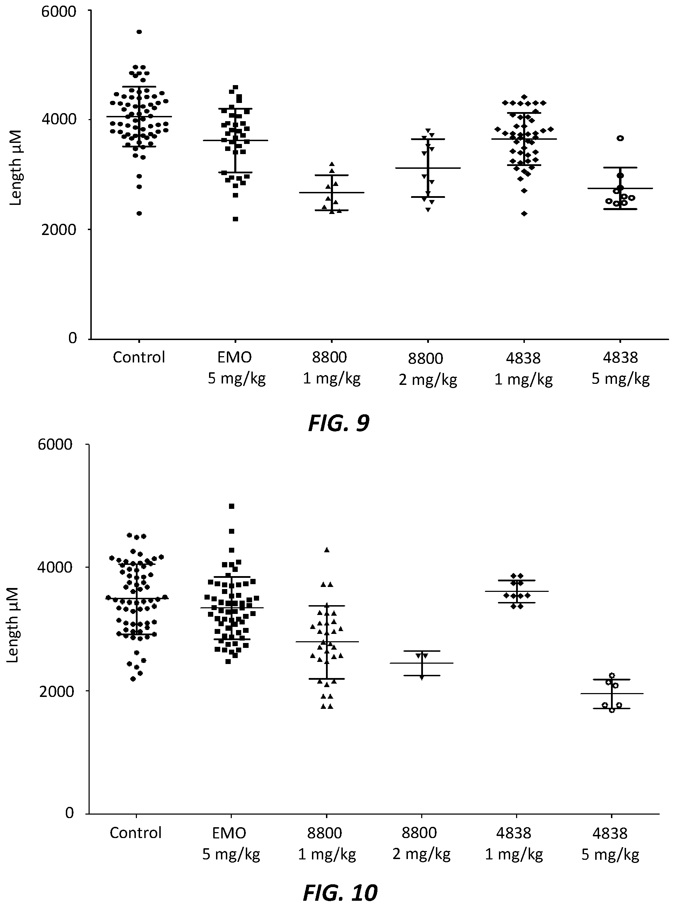

In another example, NSG mice were infected with 50 *Di* L3 (UC strain, sourced from Merial's insectary) per mouse, then treated on Days 1, 15, and 30 according to the following: EMO (5 mg/kg); 8800 (1 mg/kg); 8800 (2 mg/kg); 4838 (1 mg/kg); or 4838 (5 mg/kg). FIG. 9 is a graph showing the lengths of *Di* recovered at Days 41 to 42, and Table 12 presents the survival data, recovery data (Days 41 and 42), and summary statistics.

**Table 12. Summary data for Di-infected NSG mice treated with Control, EMO, 8800, or 4838.**

| **Control** | | | **EMO High Dose-5mg/kg** | | | **8800 Normal Dose-1mg/kg** | | | **8800 High Dose-2mg/kg** | | | **4838 Normal Dose-1mg/kg** | | | **4838 High Dose-5mg/kg** | | |
|---|---|---|---|---|---|---|---|---|---|---|---|---|---|---|---|---|---|
| # | Survival | % Rec | # | Survival | % Rec | # | Survival | % Rec | # | Survival | % Rec | # | Survival | % Rec | # | Survival | % Rec |
| 1 | 19 | 38 | 8 | 7 | 14 | 22 | 5 | 10 | 15 | 4 | 8 | 29 | 8 | 16 | 36 | 1 | 2 |
| 2 | 19 | 38 | 9 | 11 | 22 | 23 | 7 | 14 | 16 | 1 | 2 | 30 | 7 | 14 | 37 | 2 | 4 |
| 3 | 12 | 24 | 10 | 10 | 20 | 24 | 3 | 6 | 17 | 1 | 2 | 31 | 7 | 14 | 38 | 2 | 4 |
| 4 | 22 | 44 | 11 | 11 | 22 | 25 | 2 | 4 | 18 | 1 | 2 | 32 | 8 | 16 | 39 | 0 | 0 |
| 5 | 16 | 32 | 12 | 10 | 20 | 26 | 1 | 2 | 19 | 0 | 0 | 33 | 9 | 18 | 40 | 1 | 2 |
| 6 | 19 | 38 | 13 | 10 | 20 | 27 | 4 | 8 | 20 | 0 | 0 | 34 | 7 | 14 | 41 | 2 | 4 |
| 7 | 19 | 38 | 14 | 9 | 18 | 28 | 3 | 6 | 21 | 0 | 0 | 35 | 8 | 16 | 42 | 2 | 4 |
| | | | | | | | | | | | | | | | | | |
| | | | | | | | | | | | | | | | | | |
| Mean | 18 | 36 | Mean | 10 | 19 | Mean | 4 | 7 | Mean | 1 | 2 | Mean | 8 | 15 | Mean | 1 | 3 |
| SD | 3 | 6 | SD | 1 | 3 | SD | 2 | 4 | SD | 1 | 3 | SD | 1 | 2 | SD | 1 | 2 |
| | | | % Red | 47% | | % Red | 81% | | % Red | 94% | | % Red | 58% | | % Red | 92% | |
| | | | P value | 0.000 | | P value | 0.000 | | P value | 0.000 | | P value | 0.000 | | P value | 0.000 | |

In another example, NSG mice were infected with 50 *Di* L3 (UC Strain) per mouse, then treated on Days 1, 15, and 30 according to the following: EMO (1 mg/kg); 8800 (1 mg/kg); 8800 (2 mg/kg); 4838 (1 mg/kg); or 4838 (5 mg/kg). FIG. 10 is a graph showing the lengths of *Di* recovered at Days 40 to 42, and Table 13 presents the survival data, recovery data (Days 41 to 43), and summary statistics.

**Table 13. Summary data for Di-infected NSG mice treated with Control, EMO, 8800 or 4838.**

| **Control** | | | **EMO 1 mg/kg** | | | **8800 1 mg/kg** | | | **8800 2 mg/kg** | | | **4838 1 mg/kg** | | | **4838 5 mg/kg** | | |
|---|---|---|---|---|---|---|---|---|---|---|---|---|---|---|---|---|---|
| # | Survival | % Rec | # | Survival | % Rec | # | Survival | % Rec | # | Survival | % Rec | # | Survival | % Rec | # | Survival | % Rec |
| 1 | 20 | 40 | 8 | 6 | 12 | 11 | 2 | 4 | 14 | 0 | 0 | 18 | 3 | 6 | 17 | 1 | 2 |
| 2 | 14 | 28 | 9 | 15 | 30 | 12 | 3 | 6 | 15 | 1 | 2 | 19 | 1 | 2 | 35 | 2 | 4 |
| 3 | 21 | 42 | 10 | 18 | 36 | 13 | 6 | 12 | 16 | 2 | 4 | 20 | 3 | 6 | 36 | 1 | 2 |
| 4 | 15 | 30 | 33 | 9 | 18 | 29 | 5 | 10 | 25 | 1 | 2 | 21 | 5 | 10 | 37 | 1 | 2 |
| 5 | 19 | 38 | 34 | 13 | 26 | 30 | 7 | 14 | 26 | 0 | 0 | 22 | 7 | 14 | 40 | 1 | 2 |
| 6 | 24 | 48 | 38 | 9 | 18 | 31 | 5 | 10 | 27 | 3 | 6 | 23 | 4 | 8 | 41 | 0 | 0 |
| 7 | 20 | 40 | 39 | 15 | 30 | 32 | 8 | 16 | 28 | 1 | 2 | 24 | 10 | 20 | 42 | 2 | 4 |
| | | | | | | | | | | | | | | | | | |
| Mean | 19 | 38 | Mean | 12 | 24 | Mean | 5 | 10 | Mean | 1 | 2 | Mean | 5 | 9 | Mean | 1 | 2 |
| SD | 3 | 7 | SD | 4 | 9 | SD | 2 | 4 | SD | 1 | 2 | SD | 3 | 6 | SD | 1 | 1 |
| | | | % Red | 37% | | % Red | 73% | | % Red | 94% | | % Red | 75% | | % Red | 94% | |
| | | | P value | 0.000 | | P value | 0.000 | | P value | 0.000 | | P value | 0.000 | | P value | 0.000 | |

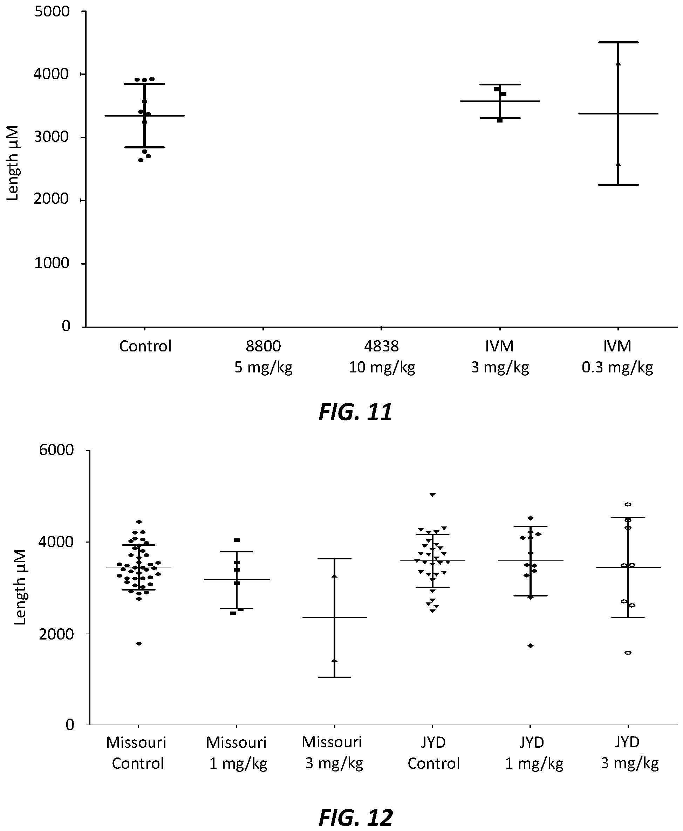

In another example, NSG mice were infected with 50 *Di* L3 per mouse, then treated on Days 1, 15, and 30 according to the following: 8800 (5 mg/kg); 4838 (10 mg/kg); IVM (3 mg/kg); or IVM (0.3 mg/kg). FIG. 11 is a graph showing the lengths of *Di* recovered at Days 41 to 42, and Table 14 presents the survival data, recovery data, and summary statistics.

**Table 14. Summary data for Di-infected NSG mice treated with Control, 8800, 4838, or IVM.**

| **Control** | | | **8800 5 mg/kg** | | | **4838 10 mg/kg** | | | **IVM 3 mg/kg** | | | **IVM 0.3 mg/kg** | | |
|---|---|---|---|---|---|---|---|---|---|---|---|---|---|---|
| # | Survival | % Rec | # | Survival | % Rec | # | Survival | % Rec | # | Survival | % Rec | # | Survival | % Rec |
| 1 | 7 | 14 | 6 | 0 | 0 | 11 | 0 | 0 | 16 | 0 | 0 | 21 | 1 | 2 |
| 2 | 9 | 18 | 7 | 0 | 0 | 12 | 0 | 0 | 17 | 1 | 2 | 22 | 0 | 0 |
| 3 | 2 | 4 | 8 | 0 | 0 | 13 | 0 | 0 | 18 | 1 | 2 | 23 | 0 | 0 |
| 4 | 8 | 16 | 9 | 0 | 0 | 14 | 0 | 0 | 19 | 0 | 0 | 24 | 0 | 0 |
| 5 | 8 | 16 | 10 | 0 | 0 | 15 | 0 | 0 | 20 | 1 | 2 | 25 | 1 | 2 |
| | | | | | | | | | | | | | | |
| | | | | | | | | | | | | | | |
| Mean | 7 | 14 | Mean | 0 | 0 | Mean | 0 | 0 | Mean | 1 | 1 | Mean | 0 | 1 |
| SD | 3 | 6 | SD | 0 | 0 | SD | 0 | 0 | SD | 1 | 1 | SD | 1 | 1 |
| | | | % Red | 100% | | % Red | 100% | | % Red | 93% | | % Red | 93% | |
| | | | P value | 0.000 | | P value | 0.000 | | P value | 0.000 | | P value | 0.000 | |

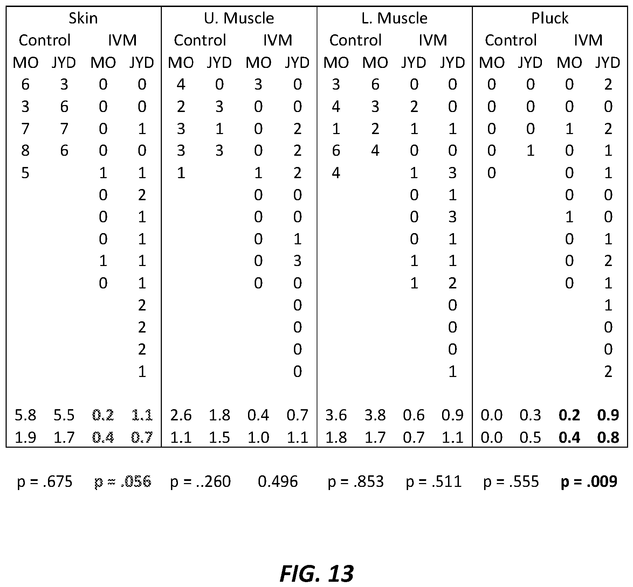

In another example, NSG mice were infected on Day 0 with 50 *Di* L3 (FR3 worms) per mouse, then treated on Days 1, 15, and 30 according to the following: MO Control; MO IVM (1 mg/kg); MO IVM (3 mg/kg); JYD Control; JYD IVM (1 mg/kg); JYD IVM (3 mg/kg); 4838 (10 mg/kg); IVM (3 mg/kg); or IVM (0.3 mg/kg). We used either the "ivermectin-susceptible" Missouri strain or the "ivermectin -resistant" JYD strain. The Missouri strain (i.e., Missouri isolate) and the JYD strain (i.e., isolate JYD-27) are described, for example, in Maclean et al. (2017) *Parasit. Vectors* 10(Suppl 2):480. The Missouri strain is also described, for example, in Bourguinat et al. (2011) Vet. Parasitol. 176(4):368-373. FIG. 12 is a graph showing the lengths of *Di* recovered at Days 41 to 42, and Table 15 presents the survival data, recovery data (Days 41 and 42), and summary statistics. FIG. 13 shows the worm recovery locations.

**Table 15. Summary data for Di-infected NSG mice treated with Control, 8800, 4838, or IVM.**

| Group 1 | | | Group 2 | | | Group 3 | | | Group 4 | | | Group 5 | | | Group 6 | | |
|---|---|---|---|---|---|---|---|---|---|---|---|---|---|---|---|---|---|
| **Missouri Control** | | | **Missouri IVM 1 mg/kg** | | | **Missouri IVM 3 mg/kg** | | | **JYD Control** | | | **JYD IVM 1 mg/kg** | | | **JYD IVM 3 mg/kg** | | |
| # | Survival | % Rec | # | Survival | % Rec | # | Survival | % Rec | # | Survival | % Rec | # | Survival | % Rec | # | Survival | % Rec |
| 1 | 13 | 26 | 6 | 3 | 6 | 11 | 0 | 0 | 16 | 9 | 18 | 23 | 6 | 12 | 21 | 2 | 4 |
| 2 | 18 | 36 | 7 | 2 | 4 | 12 | 1 | 2 | 17 | 12 | 24 | 24 | 3 | 6 | 22 | 0 | 0 |
| 3 | 22 | 44 | 8 | 2 | 4 | 13 | 0 | 0 | 18 | 10 | 20 | 25 | 7 | 14 | 29 | 7 | 14 |
| 4 | 34 | 68 | 9 | 0 | 0 | 14 | 2 | 4 | 20 | 14 | 28 | 26 | 3 | 6 | 30 | 4 | 8 |
| 5 | 20 | 40 | 10 | 3 | 6 | 15 | 1 | 2 | | | | 27 | 4 | 8 | 31 | 3 | 6 |
| | | | | | | | | | | | | 28 | 4 | 8 | 32 | 2 | 4 |
| | | | | | | | | | | | | 34 | 4 | 8 | 33 | 2 | 4 |
| | | | | | | | | | | | | | | | | | |
| | | | | | | | | | | | | | | | | | |
| Mean | 21 | 43 | Mean | 2 | 4 | Mean | 1 | 2 | Mean | 11 | 23 | Mean | 4 | 9 | Mean | 3 | 6 |
| SD | 8 | 16 | SD | 1 | 2 | SD | 1 | 2 | SD | 2 | 4 | SD | 2 | 3 | SD | 2 | 4 |
| | | | % Red: 91% | | | % Red: 95% | | | % Red | | | % Red: 61% | | | % Red: 74% | | |
| | | | P value | **0.000** | | P value | **0.000** | | P value | | | P value | **0.003** | | P value | **0.000** | |

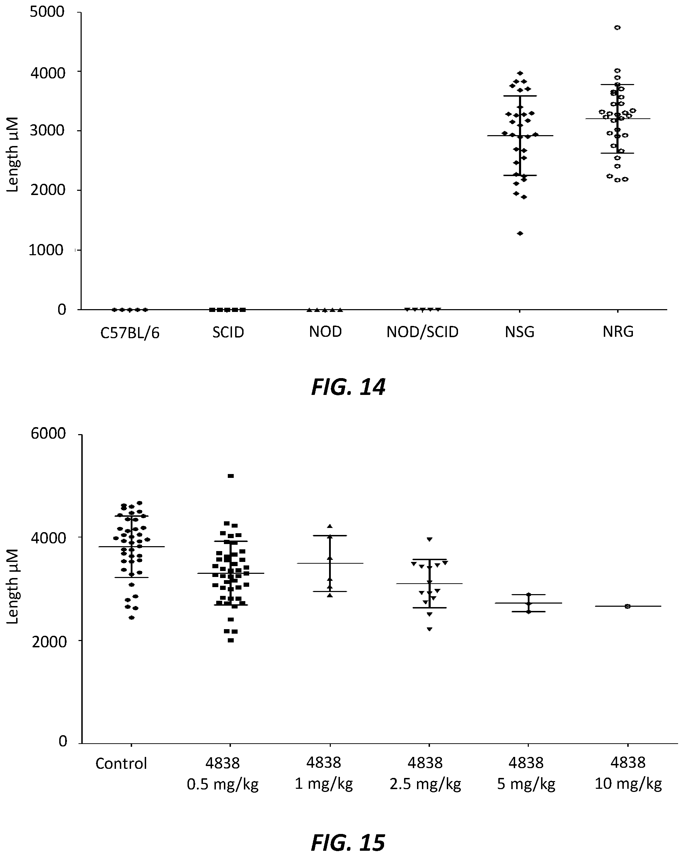

In another example, various strains of mice were infected on Day 0 with 50 *Di* L3 (FR3 worms) per mouse, but not treated subsequently. FIG. 14 is a graph showing the lengths of *Di* recovered at Days 41 to 42, and Table 16 presents the survival data, recovery data (Days 41 and 42), and summary statistics.

**Table 16. Summary data for various strains of mouse infected with FR3 Di.**

| **C57BL/6** | | | **SCID** | | | **NOD** | | | **NOD/SCID** | | | **NSG** | | | **NRG** | | |
|---|---|---|---|---|---|---|---|---|---|---|---|---|---|---|---|---|---|
| # | Survival | % Rec | # | Survival | % Rec | # | Survival | % Rec | # | Survival | % Rec | # | Survival | % Rec | # | Survival | % Rec |
| 1 | 0 | 0 | 6 | 0 | 0 | 11 | 0 | 0 | 16 | 0 | 0 | 21 | 10 | 20 | 26 | 13 | 26 |
| 2 | 0 | 0 | 7 | 0 | 0 | 12 | 0 | 0 | 17 | 0 | 0 | 22 | 17 | 34 | 27 | 8 | 16 |
| 3 | 0 | 0 | 8 | 0 | 0 | 13 | 0 | 0 | 18 | 0 | 0 | 23 | 21 | 42 | 28 | 7 | 14 |
| 4 | 0 | 0 | 9 | 0 | 0 | 14 | 0 | 0 | 19 | 0 | 0 | 24 | 12 | 24 | 29 | 10 | 20 |
| 5 | 0 | 0 | 10 | 0 | 0 | 15 | 0 | 0 | 20 | 0 | 0 | 25 | 13 | 26 | 30 | 15 | 30 |
| | | | | | | | | | | | | | | | | | |
| Mean | 0 | 0 | Mean | 0 | 0 | Mean | 0 | 0 | Mean | 0 | 0 | Mean | 15 | 29 | Mean | 11 | 21 |
| SD | 0 | 0 | SD | 0 | 0 | SD | 0 | 0 | SD | 0 | 0 | SD | 4 | 9 | SD | 3 | 7 |

In another example, NSG mice were infected on Day 0 with 50 *Di* L3 (FR3 worms) per mouse, and treated on days 1, 15, and 30 as follows: 4838 (0.5 mg/kg); 4838 (1 mg/kg); 4838 (2.5 mg/kg); 4838 (5 mg/kg); 4838 (10 mg/kg). FIG. 15 is a graph showing the lengths of *Di* recovered at Days 41 to 42, and Table 17 presents the survival data, recovery data (Days 41 and 42), and summary statistics.

**Table 17. Summary data for NSG mice infected with FR3 Di and subsequently treated with increasing doses of the 4838 depsipeptide parasiticidal agent.**

| **Control** | | | **4838 .5 mg/kg** | | | **4838 1 mg/kg** | | | **4838 2.5 mg/kg** | | | **4838 5 mg/kg** | | | **4838 10 mg/kg** | | |
|---|---|---|---|---|---|---|---|---|---|---|---|---|---|---|---|---|---|
| # | Survival | % Rec | # | Survival | % Rec | # | Survival | % Rec | # | Survival | % Rec | # | Survival | % Rec | # | Survival | % Rec |
| 1 | 14 | 28 | 8 | 6 | 12 | 15 | 5 | 10 | 22 | 2 | 4 | 29 | 0 | 0 | 36 | 0 | 0 |
| 2 | 17 | 34 | 9 | 14 | 28 | 16 | 6 | 12 | 23 | 4 | 8 | 30 | 2 | 4 | 37 | 0 | 0 |
| 3 | 15 | 30 | 10 | 11 | 22 | 17 | 5 | 10 | 24 | 1 | 2 | 31 | | 0 | 38 | 0 | 0 |
| 4 | 13 | 26 | 11 | 4 | 8 | 18 | 10 | 20 | 25 | 6 | 12 | 32 | 0 | 0 | 39 | 0 | 0 |
| 5 | 16 | 32 | 12 | 4 | 8 | 19 | 8 | 16 | 26 | 2 | 4 | 33 | 2 | 4 | 40 | 0 | 0 |
| 6 | 8 | 16 | 13 | 17 | 34 | 20 | 8 | 16 | 27 | 5 | 10 | 34 | 2 | 4 | 41 | 1 | 2 |
| 7 | 14 | 28 | 14 | 8 | 16 | 21 | 6 | 12 | 28 | 2 | 4 | 35 | 0 | 0 | 42 | 0 | 0 |
| | | | | | | | | | | | | | | | | | |
| | | | | | | | | | | | | | | | | | |
| Mean | 14 | 28 | Mean | 9 | 18 | Mean | 7 | 14 | Mean | 3 | 6 | Mean | 1 | 2 | Mean | 0 | 0.29 |
| SD | 3 | 6 | SD | 5 | 10 | SD | 2 | 4 | SD | 2 | 4 | SD | 1 | 2 | SD | 0 | 1 |
| | | | % Red: 36% | | | % Red: 50% | | | % Red: 76% | | | % Red: 93% | | | % Red: 99% | | |
| | | | P value | **0.002** | | P value | **0.000** | | P value | **0.000** | | P value | **0.000** | | P value | **0.000** | |

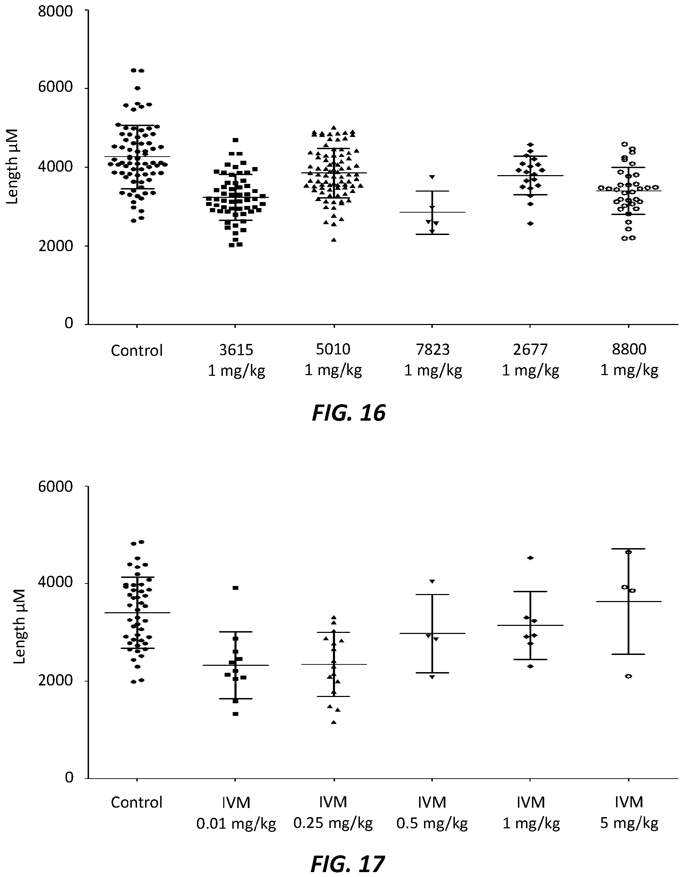

In another example, NSG mice were infected on Day 0 with 50 JYD *Di* L3 (FR3 worms) per mouse, then treated on Days 1, 15, and 30 according to the following: Control; 3615 (1 mg/kg); 5010 (1 mg/kg); 7823 (1 mg/kg); 2677 (1 mg/kg); or 8800 (1 mg/kg). FIG. 16 is a graph showing the lengths of *Di* recovered at Days 41 to 42, and Table 18 presents the survival data, recovery data (Days 41 and 42), and summary statistics.

**Table 18. Summary data for NSG mice infected with FR3 Di and subsequently treated with 3615, 5010, 7823, 2677, and 8800.**

| **Control** | | | **3615 1 mg/kg** | | | **5010 1 mg/kg** | | | **7823 1 mg/kg** | | | **2677 1 mg/kg** | | | **8800 1 mg/kg** | | |
|---|---|---|---|---|---|---|---|---|---|---|---|---|---|---|---|---|---|
| # | Survival | % Rec | # | Survival | % Rec | # | Survival | % Rec | # | Survival | % Rec | # | Survival | % Rec | # | Survival | % Rec |
| 1 | 21 | 42 | 8 | 14 | 28 | 15 | 17 | 34 | 22 | 2 | 4 | 29 | 9 | 18 | 36 | 2 | 4 |
| 2 | 23 | 46 | 9 | 18 | 36 | 16 | 15 | 30 | 23 | 1 | 2 | 30 | 8 | 16 | 37 | 13 | 26 |
| 3 | 20 | 40 | 10 | 8 | 16 | 17 | 19 | 38 | 24 | 0 | 0 | 31 | 2 | 4 | 38 | 9 | 18 |
| 4 | 24 | 48 | 11 | 12 | 24 | 18 | 12 | 24 | 25 | 1 | 2 | 32 | 3 | 6 | 39 | 6 | 12 |
| 5 | 12 | 24 | 12 | 13 | 26 | 19 | 18 | 36 | 26 | 0 | 0 | 33 | 2 | 4 | 40 | 8 | 16 |
| 6 | 25 | 50 | 13 | 15 | 30 | 20 | 12 | 24 | 27 | 0 | 0 | 34 | 2 | 4 | 41 | 6 | 12 |
| 7 | 24 | 48 | 14 | 12 | 24 | 21 | 7 | 14 | 28 | 0 | 0 | 35 | 1 | 2 | 42 | 4 | 8 |
| | | | | | | | | | | | | | | | | | |
| | | | | | | | | | | | | | | | | | |
| Mean | 21 | 43 | Mean | 13 | 26 | Mean | 14 | 29 | Mean | 1 | 1 | Mean | 4 | 8 | Mean | 7 | 14 |
| SD | 4 | 9 | SD | 3 | 6 | SD | 4 | 8 | SD | 1 | 2 | SD | 3 | 6 | SD | 4 | 7 |
| | | | % Red: 40% | | | % Red: 33% | | | % Red: 98% | | | % Red: 81% | | | % Red: 67% | | |
| | | | P value | **0.000** | | P value | **0.001** | | P value | **0.000** | | P value | **0.000** | | P value | **0.000** | |

In another example, NSG mice were infected on Day 0 with 50 JYD *Di* L3 (FR3 worms) per mouse, then treated on Days 1, 15, and 30 according to the following: Control; IVM (0.01 mg/kg); IVM (0.25 mg/kg); IVM (0.5 mg/kg); IVM (1 mg/kg); or IVM (5 mg/kg). FIG. 17 is a graph showing the lengths of *Di* recovered at Days 40 to 42, and Table 19 presents the survival data, recovery data (Days 40-42), and summary statistics.

**Table 19. Summary data for NSG mice infected with FR3 Di and subsequently treated with different doses of IVM.**

| **Control** | | | **IVM 0.01 mg/kg** | | | **IVM 0.25mg/kg** | | | **IVM 0.5 mg/kg** | | | **IVM 1 mg/kg** | | | **IVM 5 mg/kg** | | |
|---|---|---|---|---|---|---|---|---|---|---|---|---|---|---|---|---|---|
| # | Survival | % Rec | # | Survival | % Rec | # | Survival | % Rec | # | Survival | % Rec | # | Survival | % Rec | # | Survival | % Rec |
| 1 | 8 | 16 | 8 | 3 | 6 | 15 | 2 | 4 | 22 | 0 | 0 | 29 | 1 | 2 | 36 | 0 | 0 |
| 2 | 9 | 18 | 9 | 4 | 8 | 16 | 0 | 0 | 23 | 4 | 8 | 30 | 1 | 2 | 37 | 1 | 2 |
| 3 | 10 | 20 | 10 | 3 | 6 | 17 | 3 | 6 | 24 | 1 | 2 | 31 | 4 | 8 | 38 | 2 | 4 |
| 4 | 10 | 20 | 11 | 2 | 4 | 18 | 2 | 4 | 25 | 1 | 2 | 32 | 1 | 2 | 39 | 3 | 6 |
| 5 | 2 | 4 | 12 | 1 | 2 | 19 | 7 | 14 | 26 | 0 | 0 | 33 | 2 | 4 | 40 | 0 | 0 |
| 6 | 12 | 24 | 13 | 3 | 6 | 20 | 5 | 10 | 27 | 0 | 0 | 34 | 0 | 0 | 41 | 0 | 0 |
| 7 | 13 | 26 | 14 | 0 | 0 | 21 | 5 | 10 | 28 | 2 | 4 | 35 | 0 | 0 | 42 | 0 | 0 |
| | | | | | | | | | | | | | | | | | |
| | | | | | | | | | | | | | | | | | |
| Mean | 9 | 18 | Mean | 2 | 5 | Mean | 3 | 7 | Mean | 1 | 2 | Mean | 1 | 3 | Mean | 1 | 2 |
| SD | 4 | 7 | SD | 1 | 3 | SD | 2 | 5 | SD | 1 | 3 | SD | 1 | 3 | SD | 1 | 2 |
| | | | % Red: 72% | | | % Red: 61% | | | % Red: 89% | | | % Red: 83% | | | % Red: 89% | | |
| | | | P value | **0.000** | | P value | **0.000** | | P value | **0.000** | | P value | **0.000** | | P value | **0.000** | |

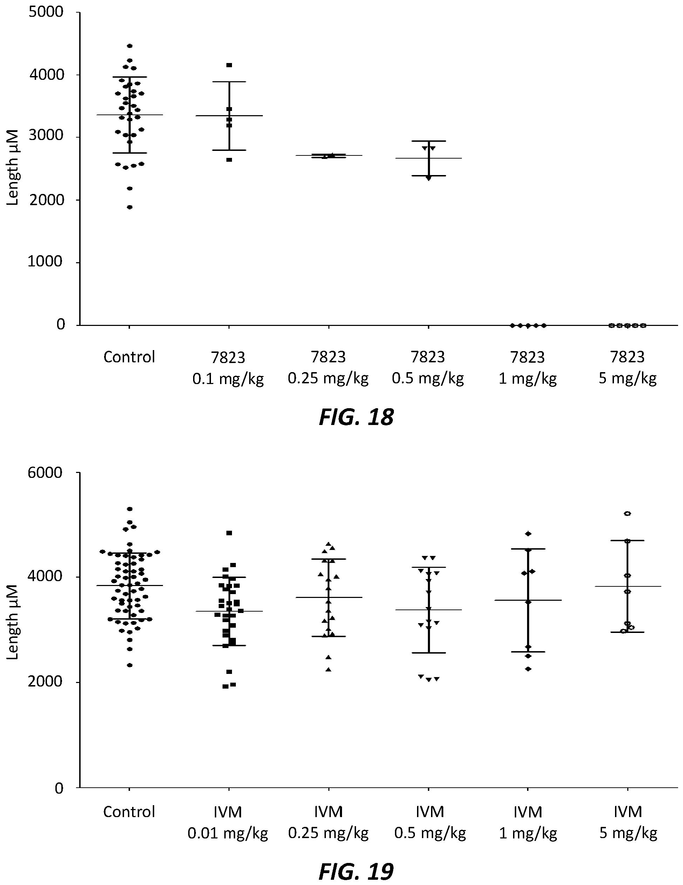

In another example, NSG mice were infected on Day 0 with 50 JYD *Di* L3 (FR3 worms) per mouse, then treated on Days 1, 15, and 30 according to the following: Control; 7823 (0.1 mg/kg); 7823 (0.25 mg/kg); 7823 (0.5 mg/kg); 7823 (1 mg/kg); or 7823 (5 mg/kg). FIG. 18 is a graph showing the lengths of *Di* recovered at Days 40 to 42, and Table 20 presents the survival data, recovery data (Days 40-42), and summary statistics.

**Table 20. Summary data for NSG mice infected with FR3 Di and subsequently treated with different doses of 7823.**

| **Control** | | | **7823 0.1 mg/kg** | | | **7823 0.25 mg/kg** | | | **7823 0.5 mg/kg** | | | **7823 1.0 mg/kg** | | | **7823 5.0 mg/kg** | | |
|---|---|---|---|---|---|---|---|---|---|---|---|---|---|---|---|---|---|
| # | Survival | % Rec | # | Survival | % Rec | # | Survival | % Rec | # | Survival | % Rec | # | Survival | % Rec | # | Survival | % Rec |
| 1 | 11 | 22 | 6 | 4 | 8 | 11 | 2 | 4 | 16 | 0 | 0 | 21 | 0 | 0 | 26 | 0 | 0 |
| 2 | 9 | 18 | 7 | 1 | 2 | 12 | 0 | 0 | 17 | 2 | 4 | 22 | 0 | 0 | 27 | 0 | 0 |
| 3 | 14 | 28 | 8 | 3 | 6 | 13 | 0 | 0 | 18 | 1 | 2 | 23 | 0 | 0 | 28 | 0 | 0 |
| 4 | 11 | 22 | 9 | 3 | 6 | 14 | 2 | 4 | 19 | 0 | 0 | 24 | 0 | 0 | 29 | 0 | 0 |
| 5 | 13 | 26 | 10 | 0 | 0 | 15 | 0 | 0 | 20 | 4 | 8 | 25 | 0 | 0 | 30 | 0 | 0 |
| | | | | | | | | | | | | | | | | | |
| | | | | | | | | | | | | | | | | | |
| Mean | 12 | 23 | Mean | 2 | 4 | Mean | 1 | 2 | Mean | 1 | 3 | Mean | 0 | 0 | Mean | 0 | 0 |
| SD | 2 | 4 | SD | 2 | 3 | SD | 1 | 2 | SD | 2 | 3 | SD | 0 | 0 | SD | 0 | 0 |
| | | | % Red: 83% | | | % Red: 91% | | | % Red: 87% | | | % Red: 100% | | | % Red: 100% | | |
| | | | P value | **0.000** | | P value | **0.000** | | P value | **0.000** | | P value | **0.000** | | P value | **0.000** | |

In another example, NSG mice were infected on Day 0 with 50 JYD *Di* L3 (FR3 worms) per mouse, then treated on Days 1, 15, and 30 according to the following: Control; IVM (0.01 mg/kg); IVM (0.25 mg/kg); IVM (0.5 mg/kg); IVM (1 mg/kg); or IVM (5 mg/kg). FIG. 19 is a graph showing the lengths of *Di* recovered at Days 40 to 42, and Table 21 presents the survival data, recovery data (Days 40-42), and summary statistics.

**Table 21. Summary data for NSG mice infected with FR3 Di and subsequently treated with different doses of IVM.**

| **Control** | | | **IVM 0.01 mg/kg** | | | **IVM 0.25mg/kg** | | | **IVM 0.5 mg/kg** | | | **IVM 1 mg/kg** | | | **IVM 5 mg/kg** | | |
|---|---|---|---|---|---|---|---|---|---|---|---|---|---|---|---|---|---|
| # | Survival | % Rec | # | Survival | % Rec | # | Survival | % Rec | # | Survival | % Rec | # | Survival | % Rec | # | Survival | % Rec |
| 1 | 14 | 28 | 8 | 6 | 12 | 15 | 0 | 0 | 22 | 2 | 4 | 29 | 3 | 6 | 36 | 3 | 6 |
| 2 | 8 | 16 | 9 | 4 | 8 | 16 | 3 | 6 | 23 | 6 | 12 | 30 | 3 | 6 | 37 | 2 | 4 |
| 3 | 11 | 22 | 10 | 8 | 16 | 17 | 2 | 4 | 24 | 1 | 2 | 31 | 0 | 0 | 38 | 2 | 4 |
| 4 | 15 | 30 | 11 | 7 | 14 | 18 | 5 | 10 | 25 | 3 | 6 | 32 | 1 | 2 | 39 | 0 | 0 |
| 43 | 18 | 36 | 12 | 9 | 18 | 19 | 4 | 8 | 26 | 0 | 0 | 33 | 2 | 4 | 40 | 3 | 6 |
| 5 | 10 | 20 | 13 | 5 | 10 | 20 | 4 | 8 | 27 | 6 | 12 | 34 | 1 | 2 | 41 | 2 | 4 |
| 6 | 13 | 26 | 14 | 7 | 14 | 21 | 5 | 10 | 28 | 4 | 8 | 35 | 1 | 2 | 42 | 4 | 8 |
| 7 | 6 | 12 | | | | | | | | | | | | | | | |
| | | | | | | | | | | | | | | | | | |
| Mean | 12 | 24 | Mean | 7 | 13 | Mean | 3 | 7 | Mean | 3 | 6 | Mean | 2 | 3 | Mean | 2 | 5 |
| SD | 4 | 8 | SD | 2 | 3 | SD | 2 | 4 | SD | 2 | 5 | SD | 1 | 2 | SD | 1 | 3 |
| | | | % Red: 46% | | | % Red: 71% | | | % Red: 75% | | | % Red: 88% | | | % Red: 79% | | |
| | | | P value | 0.000 | | P value | 0.000 | | P value | 0.000 | | P value | 0.000 | | P value | 0.000 | |

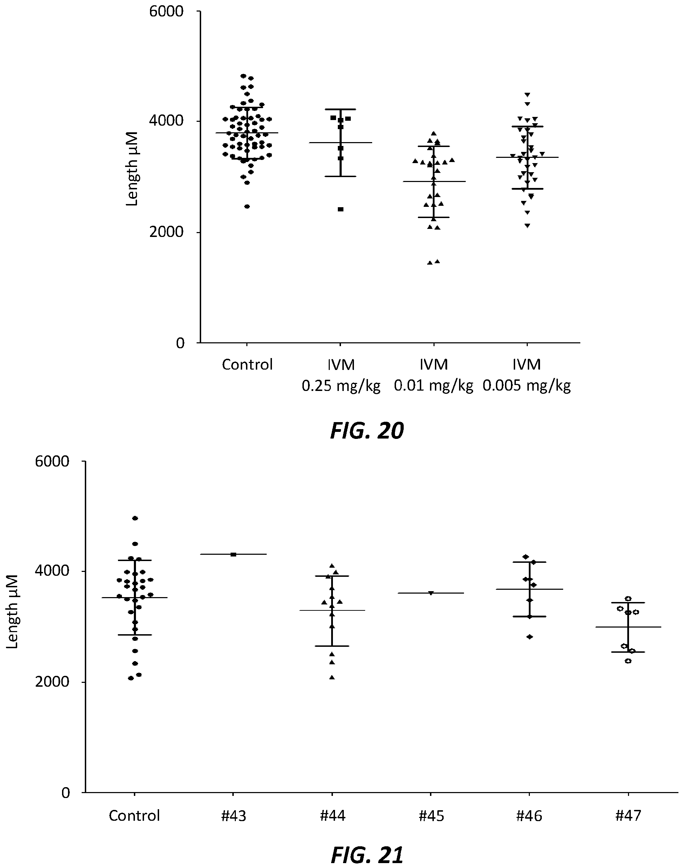

In another example, NSG mice were infected on Day 0 with 50 JYD *Di* L3 (FR3 worms) per mouse, then treated on Days 1, 15, and 30 according to the following: Control; IVM (0.25 mg/kg); IVM (0.01 mg/kg); or IVM (0.005 mg/kg). FIG. 20 is a graph showing the lengths of *Di* recovered at Days 40 to 42, and Table 22 presents the survival data, recovery data (Days 40-42), and summary statistics.

**Table 22. Summary data for NSG mice infected with FR3 Di and subsequently treated with IVM.**

| **Control** | | | **IVM 0.25 mg/kg** | | | **IVM 0.01mg/kg** | | | **IVM 0.005 mg/kg** | | |
|---|---|---|---|---|---|---|---|---|---|---|---|
| # | Survival | % Rec | # | Survival | % Rec | # | Survival | % Rec | # | Survival | % Rec |
| 1 | 14 | 28 | 8 | 6 | 12 | 15 | 6 | 12 | 22 | 6 | 12 |
| 2 | 16 | 32 | 9 | 4 | 8 | 16 | 3 | 6 | 23 | 7 | 14 |
| 3 | 15 | 30 | 10 | 0 | 0 | 17 | 2 | 4 | 24 | 7 | 14 |
| 4 | 15 | 30 | 11 | 1 | 2 | 18 | 4 | 8 | 25 | 6 | 12 |
| 5 | 15 | 30 | 12 | 0 | 0 | 19 | 2 | 4 | 26 | 5 | 10 |
| 6 | 6 | 12 | 13 | 3 | 6 | 20 | 2 | 4 | 27 | 5 | 10 |
| 7 | 10 | 20 | 14 | 2 | 4 | 21 | 6 | 12 | 28 | 6 | 12 |
| | | | | | | | | | | | |
| | | | | | | | | | | | |
| Mean | 13 | 26 | Mean | 2 | 5 | Mean | 4 | 7 | Mean | 6 | 12 |
| SD | 4 | 7 | SD | 2 | 4 | SD | 2 | 4 | SD | 1 | 2 |
| | | | % Red: 81% | | | % Red: 73% | | | % Red: 54% | | |
| | | | P value | **0.000** | | P value | 0.000 | | P value | **0.000** | |

A single experiment was performed in which NSG mice were infected with approximately 200,000 JYD strain *D. immitis* microfilariae by retro-orbital infection. Microfilariae were consistently recovered from the retro-orbital blood at 4 weeks post-infection. *See* Table 23. Through this experiment we demonstrated that the NSG mouse was also a suitable host for the microfilarial stage of *D. immitis.*

**Table 23. Infection of NSG mice with Di microfilariae.**

| **Mouse ID** | **Mf Count/100 µL Blood** | | |
|---|---|---|---|
| | **2 Week** | **4 Week** | **6 Week** |
| 1R | 7 | 3 | 0 |
| 1L | 10 | 4 | X |
| RL | 12 | 4 | 1 |
| NT | 6 | 2 | 0 |
| 2R2L | 12 | 10 | 0 |
| 2R | 8 | 6 | 2 |

### Example 2 - Infection of Immunocompromised Mice with Ivermectin-Resistant (IVM^{R}) Strains of D. immitis to Screen for Novel Compounds to Address Resistant Strains

NSG mice infected with resistant HW are exposed to novel compounds to determine the efficacy of these compounds in killing resistant parasites. The source of the novel compounds may be from pharmaceutical libraries and they would be administered to the mice in a range of doses to measure efficacy and killing IVM-resistant and IVM-susceptible strains of HW.

One such strain that is resistant (i.e., less susceptible) to ivermectin is the JYD strain described above. The JYD strain (i.e., isolate JYD-27) is described, for example, in Maclean et al. (2017) Parasit. Vectors 10(Suppl 2):480. Experiments in which compounds were tested against the JYD strain in NSG immunocompromised mice are described in Example 1. *See, e.g*., FIGS. 12, 13, and 16-20 and Tables 15 and 18-22. *See also* FIG. 21 and Table 25 in Example 3.

### Example 3 - Development and Validation of an Immunocompetent, Intermediate in Vivo Model of Canine Heartworm Infection with Engrafted Canine Immune Cells

We performed an initial experiment using peripheral blood mononuclear cells (PBMCs) purified from whole blood. Canine PBMCs were isolated from 10 mL of whole blood using a Ficoll gradient per manufacturer's instructions for the isolation of human PBMC. Six NSG mice were injected via intraperitoneal (IP) injection with 5.0 × 10⁶ PBMCs from a single donor animal and screened by flow cytometry 4 and 8 weeks later. At 4 weeks post-engraftment, all mice were positive for canine B cells and CD4 and CD8 T cells, along with a number of other canine CD45⁺ cells present that the panel did not identify (Table 24 and data not shown). Percent engraftment was calculated by the total canine CD45+ cells within the lymphocyte/granulocyte gate, which is determined based on cell size and cell granularity. At 8 weeks post-engraftment 3 mice had succumbed to graft vs host disease (GVHD), and of the remaining 3 mice, only one had significant levels of canine PBMCs remaining. This study shows that NSG mice are capable of supporting canine immune cells and should provide a suitable platform for the engraftment of CD34⁺ stem cells.

**Table 24. Canine cell levels in NSG mice at 3-weeks post-engraftment.**

| **CD45+** | **CD3+** | **CD4+** | **CD8+** |
|---|---|---|---|
| 29 ± 20% | 8 ± 7% | 16 ± 14% | 3 ± 3% |

To further confirm the initial PBMC findings, PBMCs were purified from 100 mL of canine whole blood. Fifteen NSG mice were injected with either 5 × 10⁶ (10 mice) or 10 × 10⁶ (5 mice) PBMCs from a donor animal. Mice receiving 5 × 10⁶ cells had an average canine CD45⁺ engraftment of 16 ± 16%, and the mice receiving 10 × 10⁶ cells had an average engraftment of 28 ± 13%. All mice succumbed to GVHD between 3 and 6 weeks post-engraftment.

Next, two groups of CD34+ stem cells were purified from two different canine umbilical cord blood collections. Canine CD34⁺ stem cells were purified using a biotin labeled anti-canine CD34⁺ antibody, in conjunction with a magnetic assisted cell sorting kit (MACS) that specifically recognized the biotin labeled antibody. Due to the small amount of cord blood obtained from a puppy, it is impossible to purify stem cells from a single donor. Two mixed-donor purified CD34⁺ cells batches were purified with cell counts of 2.65 × 10⁵ and 2.2 × 10⁵ and cell purities of 74 and 77%, respectively. 50,000 canine CD34+ cells isolated from umbilical cord blood were then engrafted into five 48-hour-old NSG pups irradiated with 150 RAD. Their engraftment status was verified at 8 weeks. Based on our experience with humanized mice, it was predicted that the mice would be optimally engrafted at around 12 weeks. At 12 weeks post-engraftment, the 5 mice had average canine CD45⁺ cell levels of 58 ± 6%. The caninized mice (CnMice) were then infected with 50 JYD *Di* L3 about one week later (Day 0) in conjunction with an NSG mouse infection. The mice were necropsied on Days 40 through 42 post-infection. It was concluded that there was no difference in the parasite survival and growth in the CnMice vs. the NSG mice. FIG. 21 is a graph showing the lengths of *Di* recovered at Days 40 to 42 in the canine mice, and Table 25 presents the survival and recovery data (Days 40-42). There was no difference in the parasite survival and growth in the CnMice vs. the NSG mice.

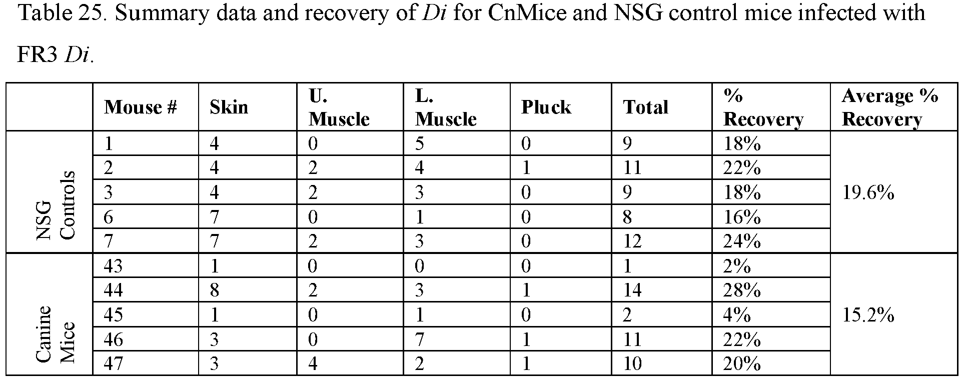

Due to low numbers of CD34⁺ stem cells that are available from canine cord blood, we attempted to isolate stem cells from bone marrow collected from newborn puppies. CD34⁺ stem cells were isolated from bone marrow using a similar method to the cord blood isolation protocol. Using bone marrow from three mixed donor dogs, and following MACS beads and column isolation, we were able to isolate 4.4 × 10⁵ CD34⁺ cells. These cells were engrafted into 7 NSG mice. At 12 weeks post engraftment the mice had canine CD45⁺ cell levels of 21 ± 19% of the total lymphocyte/granulocyte population.

CnMice of bone marrow CD34⁺ cell origin were then infected with 100 JYD L3 *Di.* These mice were necropsied on Days 40 through 42 post-infection. The recovery data is shown in Table 26. No significant difference was seen between the two groups. The absence of significant difference in parasite recoveries between the controls and either CnMouse model suggests these are viable models to begin to understand the relationship between the canine immune system and the development of *D. immitis.*

**Table 26. Summary data and recovery of Di for CnMice and NSG control mice infected with FR3 Di.**

| **Group** | **Mouse #** | **Skin** | **U. Muscle** | **L. Muscle** | **Pluck** | **Total** | **% Recovery** | **Average % Recovery** |
|---|---|---|---|---|---|---|---|---|
| Control NSG Mice | 1 | 18 | 9 | 10 | 0 | 37 | 37% | 33.1% |
| | 2 | 9 | 10 | 9 | 0 | 28 | 28% | |
| | 3 | 8 | 7 | 19 | 0 | 34 | 34% | |
| | 4 | 11 | 3 | 8 | 0 | 22 | 22% | |
| | 5 | 20 | 4 | 15 | 1 | 40 | 40% | |
| | 6 | 14 | 4 | 19 | 0 | 37 | 37% | |
| | 7 | 7 | 5 | 13 | 0 | 25 | 25% | |
| | 8 | 10 | 18 | 10 | 0 | 38 | 38% | |
| | 9 | 12 | 5 | 11 | 0 | 28 | 28% | |
| | 10 | 18 | 7 | 17 | 0 | 42 | 42% | |
| CnMice | 11 | 11 | 10 | 12 | 5 | 38 | 38% | 36.3% |
| | 12 | 14 | 6 | 1 | 3 | 24 | 24% | |
| | 13 | 25 | 9 | 7 | 0 | 41 | 41% | |
| | 14 | 12 | 6 | 15 | 1 | 34 | 34% | |
| | 15 | 22 | 18 | 21 | 0 | 61 | 61% | |
| | 16 | 13 | 2 | 6 | 1 | 22 | 22% | |
| | 17 | 15 | 7 | 11 | 1 | 34 | 34% | |

### Example 4 - Development and Validation of an In Vivo Model of Gastrointestinal Nematode Infection

*Haemonchus contortus* is a gastrointestinal worm that infects mainly sheep and goats, but also cattle. These worms do not affect dogs and cats. The disease caused by *Haemonchus* worms is called haemonchosis. *Haemonchus* worms have a direct life cycle (there are no intermediate hosts involved). Adult females lay eggs in the stomach of the host that are shed with the feces. Once in the environment, the eggs release the L1-larvae that complete development to infective L3-larvae in about 4 to 7 days. Livestock becomes infected after ingesting infective larvae. These larvae enter the pit of gastric glands and feed on blood flowing out of the lesions they cause to the stomach's lining. Later, they complete development to adult worms and females start producing eggs.

In a pilot experiment, mice were infected with either 500 or 1,000 larvae of *H. contortus.* In addition, some of the mice were further immune compromised by treatment with the steroid methylprednisolone acetate. Following infection, mice showed a drop in weight of 10-20%, and then either regained or remained the same throughout the rest of the experiment. Mice were necropsied periodically over 8 weeks, looking in the stomach for adult worms and in the intestinal contents for eggs. It was discovered that *H. contortus* larvae develop to adults that contained blood within their intestines. Importantly, eggs were seen in the intestinal contents at 28 days post-infection. Steroid treatment to further compromise the immune response did not increase worm recoveries.

NSG mice were pre-treated with methylprednisolone acetate (MPA) one week prior to infection. *H. contortus* L3 were both agar cleaned and/or cleaned with NCTC/IMDM (NI) media with antibiotics. 500 and 1000 L3 were dosed to control and MPA-treated mice by oral gavage. At dosing, mice were weighed and given a general health score. Mice were checked biweekly for health scores and weighed once a week at MPA dosing (MPA injections are weekly). Forty total mice were infected: 20 with MPA and 20 controls, with 10 in each group for doses of 500 and 1000.

The day prior to necropsy, mice were fasted. Stomachs and small intestines were then removed. The stomach was cut open and hung in a 40 mL conical in PBS. The small intestines were cut open and hung around a paper clip in a 50 mL conical in PBS. The tissues were then incubated at 50°C for 2.5 hours, after which the tissues were discarded. The tubes were shaken and poured through a 200 µm mesh strainer and rinsed. Worms were collected by back washing off of the strainer and were counted. Worms were saved fixed with glycerol/alcohol to be measured.

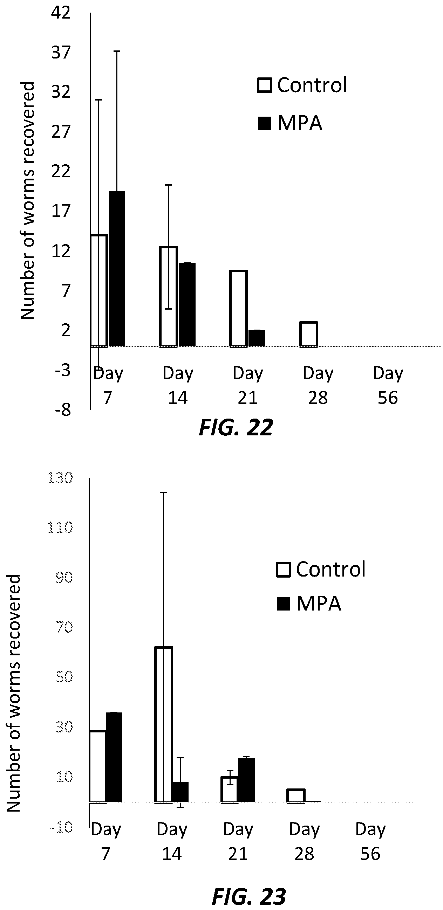

*H. contortus* recovery counts for the 500 and 1000 starting infections are shown in Tables 27 and 28, respectively, and in FIGS. 22 and 23, respectively. "Recovery" refers to the number of *Hc* worms recovered from a mouse. Eggs collected from fecal content between Days 23-31 post-infection (day 28 post-infection) in four of the infected mice are shown in Table 29.

**Table 27. Hc recovery counts from mice with starting infection of 500 Hc L3.**

| | **Control** | | | **MPA** | | |
|---|---|---|---|---|---|---|
| | **Mouse ID** | **Recovery** | **Average** | **Mouse ID** | **Recovery** | **Average** |
| 7 days PI | 7 | 26 | 14 | 1 | 36 | 19.5 |
| | 8 | 2 | | 3 | 3 | |
| 14 Days PI | 15 | 0 | 12.5 | 12 | 2 | 10.5 |
| | 16 | 25 | | 11 | 19 | |
| 21 Days PI | 22 | 4 | 9.5 | 18 | 2 | 2 |
| | 23 | 15 | | 19 | 2 | |
| 28 Days PI | 30 | 3 | 3 | 25 | 0 | 0 |
| | 31* | 3 | | 27 | 0 | |
| 56 Days PI | 39 | 0 | 0 | 33 | 0 | 0 |
| | 40 | 0 | | 34 | 0 | |

| | | | | | | |
|---|---|---|---|---|---|---|
| * indicates egg collection | | | | | | |

**Table 28. Hc recovery counts from mice with starting infection of 1000 Hc L3.**

| | **Control** | | | **MPA** | | |
|---|---|---|---|---|---|---|
| | **Mouse ID** | **Recovery** | **Average** | **Mouse ID** | **Recovery** | **Average** |
| 7 days PI | 5 | 33 | 28.5 | 2 | 24 | 36 |
| | 6 | 24 | | 4 | 48 | |
| 14 Days PI | 13 | 18 | 62 | 10 | 1 | 8 |
| | 14 | 106 | | 9 | 15 | |
| 21 Days PI | 21 | 11 | 10 | 17 | 15 | 17.5 |
| | 24 | 9 | | 20 | 20 | |
| 28 Days PI | 29* | 7 | 5 | 26* | 0 | 0.5 |
| | 32* | 3 | | 28 | 1 | |
| 56 Days PI | 37 | 0 | 0 | 35 | 0 | 0 |
| | 38 | 0 | | 36 | 0 | |

| | | | | | | |
|---|---|---|---|---|---|---|
| * indicates egg collection | | | | | | |

**Table 29. Eggs found at week four in four mice infected with either 500 or 1000 Hc L3.**

| **Mouse ID** | **Experiment** | **# Eggs Found** |
|---|---|---|
| 26 | 1000 infected, MPA | 2 |
| 29 | 1000 infected, control | 9 |
| 31 | 500 infected, control | 41 |
| 32 | 1000 infected, control | 8 |

Mice were checked biweekly over the infection period for a visual health screen and always scored 0 on our health assessment (score of 8 is an indicator for euthanasia). Mice were weighed weekly to monitor for excessive weight loss (data not shown).

In a second experiment, 43 NSG mice were infected with 1000 L3 as described above. *H*. *contortus* recovery counts at various time points are shown in Table 30. Eggs were recovered from the mice 26 days post-infection and were incubated at 26°C for 18 days. Twenty L1 larvae were recovered.

**Table 30. Hc recovery counts from mice with starting infection of 1000 Hc L3.**

| **Time Point** | **Number of Mice Infected** | **Average Recovery** |
|---|---|---|
| 15 Days PI | 6 | 15 |
| 23 Days PI | 6 | 4 |
| 28 Days PI | 22 | 2 |
| 31 Days PI | 8 | 1 |

*Strongyloides stercoralis (threadworm)* is a human pathogenic parasitic roundworm that causes the disease strongyloidiasis. The adult parasitic stage lives in tunnels in the mucosa of the small intestine. *S. stercoralis* has been reported in other mammals, including cats and dogs. *S*. *stercoralis* infects an estimated 100 million people. Its ability to autoinfect its host enables it to persist for decades undetected and to progress to a potentially fatal hyperinfection that often is induced by glucocorticoid treatment. The mouse model described herein recapitulates all forms of human strongyloidiasis. Treatment with methylprednisolone acetate (MPA), a synthetic glucocorticoid, induces hyperinfection in NSG mice.

NSG mice infected by subcutaneous injection with 5000 *S. stercoralis* L3 larvae, either with or without MPA. Mice were treated with 2.5 mg/kg EMO or 3 mg/kg IVM on days 1, 15, and 30 post-injection. Adult worms, L1 larvae, or L3 larvae were counted in the mice at 6 weeks post-injection. As shown in Tables 31 and 32, EMO and IVM clearly controlled the intestinal infection, eliminating adult worms and L1 larvae, respectively, in models of both normal, chronic infection (control) and hyperinfection (MPA). As shown in Table 33, EVO and IVM reduced the circulating L3 in models of both normal, chronic infection (control) and hyperinfection (MPA), but there were still some circulating L3 in the body.

**Table 31. Adult S. stercoralis recovery counts from mice at 6 weeks post-injection.**

| | **Control** | **MPA** | **EMO Control** | **EMO / MPA** | **IVM Control** | **IVM / MPA** |
|---|---|---|---|---|---|---|
| | 16 | 437 | 0 | 0 | 0 | 0 |
| | 2 | 581 | 0 | 0 | 0 | 0 |
| | 17 | 447 | 0 | 0 | 0 | 0 |
| | 10 | 888 | 0 | 0 | 0 | 0 |
| | 10 | 235 | 1 | 0 | 0 | 0 |
| | | 34 | | 0 | | 0 |
| | | | | 0 | | 0 |
| | | | | | | 0 |
| **mean** | 11 | 437 | 0.2 | 0 | 0 | 0 |
| **SD** | 6 | 292 | 0.4 | 0 | 0.0 | 0 |

**Table 32. L1 S. stercoralis recovery counts from mice at 6 weeks post-injection.**

| | **Control** | **MPA** | **EMO Control** | **EMO / MPA** | **IVM Control** | **IVM / MPA** |
|---|---|---|---|---|---|---|
| | 32 | 8456 | 0 | 0 | 0 | 0 |
| | 4 | 9181 | 0 | 0 | 0 | 0 |
| | 15 | 3464 | 0 | 0 | 0 | 0 |
| | 46 | 670 | 0 | 0 | 0 | 0 |
| | 64 | 86 | 0 | 0 | 0 | 0 |
| | | 24 | | 0 | | 0 |
| | | | | 0 | | 0 |
| | | | | | | 0 |
| **mean** | 32 | 3647 | 0 | 0 | 0 | 0 |
| **SD** | 24 | 4206 | 0.0 | 0 | 0.0 | 0 |

**Table 33. L3 S. stercoralis recovery counts from mice at 6 weeks post-injection.**

| | **Control** | **MPA** | **EMO Control** | **EMO / MPA** | **IVM Control** | **IVM / MPA** |
|---|---|---|---|---|---|---|
| | 496 | 2760 | 8 | 102 | 9 | 25 |
| | 202 | 1766 | 0 | 130 | 26 | 16 |
| | 218 | 632 | 1 | 39 | 210 | 40 |
| | 407 | 1015 | 5 | 18 | 2 | 38 |
| | 497 | 275 | 5 | 4 | 6 | 19 |
| | | 31 | | 123 | | 24 |
| | | | | 228 | | 4 |
| | | | | | | 13 |
| **mean** | 364 | 1080 | 4 | 92 | 51 | 22 |
| **SD** | 145 | 1024 | 3 | 79 | 90 | 12 |

## Claims

1. A method of determining the effectiveness of a compound or combination of compounds for treating or preventing a parasitic worm infection, comprising:
(a) infecting an immunocompromised rodent with a specific number of parasitic worm larvae;
(b) treating the infected rodent with the compound or combination of compounds to be tested; and
(c) necropsying the rodent and determining the number of parasitic worms, optionally adult parasitic worms, present in a rodent tissue;
wherein the rodent is an NOD scid IL2 receptor gamma chain knockout mouse (NSG) (NOD-SCID-IL2Ry -/-) or an NRG (NOD-Rag1^{null}IL2rg^{null}) immunodeficient mouse; and wherein the parasitic worm is a nematode and the parasitic worm is *Dirofilaria immitis, Strongyloides stercoralis* or *Haemonchus contortus.*

2. The method of claim 1, further comprising:
(d) infecting a control immunocompromised rodent with a specific number of parasitic worm larvae;
(e) necropsying the control immunocompromised rodent and determining the number of worms, optionally adult parasitic worms, present in a rodent tissue; and
(f) comparing the number of worms in the rodent tissue in step (c) with the number of worms in the rodent tissue in step (e).

3. A method of determining the number of parasitic worms in one or more isolated tissue sample(s) of an immunocompromised rodent, preferably a necropsied immunocompromised rodent, infected with a specific number of parasitic worm larvae and treated with a compound or combination of compounds to be tested, wherein the method comprises determining the number of parasitic worms, optionally adult parasitic worms, present in the isolated tissue sample(s); wherein the parasitic worm is a nematode and the parasitic worm is *Dirofilaria immitis, Strongyloides stercoralis* or *Haemonchus contortus* and wherein the rodent is an NOD scid IL2 receptor gamma chain knockout mouse (NSG) (NOD-SCID-IL2Ry -/-) or an NRG (NOD-Rag1^{null}IL2rg^{null}) immunodeficient mouse.

4. The method of claim 3, further comprising determining the number of parasitic worms in one or more isolated tissue sample(s) of an untreated immunocompromised rodent, preferably a necropsied immunocompromised rodent, infected with a specific number of parasitic worm larvae, wherein the method comprises determining the number of parasitic worms, optionally adult parasitic worms, present in the isolated tissue sample(s) from the untreated immunocompromised rodent, and comparing the number of parasitic worms present in the isolated tissue sample(s) from the untreated immunocompromised rodent with the number of parasitic worms present in the isolated tissue sample(s) from the treated immunocompromised rodent.

5. The method of any one of claims 1 to 4, wherein the parasitic worm larvae are selected from the group consisting of microfilariae, L3 larvae, and L4 larvae.

6. The method of any one of claims 1 to 5, wherein the immunocompromised rodent does not comprise engrafted immune cells from a different species.

7. The method of any one of claims 1 to 6, wherein the immunocompromised rodent comprises engrafted immune cells from a definitive host of the parasitic worm, optionally wherein the engrafted immune cells are canine immune cells.

8. The method of any one claims 1 to 7, further comprising comparing the parasiticidal efficacy of the compound or combination of compounds against the parasiticidal efficacy of either or both a macrocyclic lactone (ML) parasiticidal compound, preferably wherein the ML compound is selected from avermectin, ivermectin, doramectin, abamectin, milbemycin and moxidectin, and/or a depsipeptide parasiticidal compound, preferably wherein the depsipeptide parasiticidal compound is emodepside.

9. The method of claim 1 or 3, wherein the immunocompromised rodent is an NSG or an NRG mouse, and wherein the parasitic worm is *Dirofilaria immitis;* or wherein the immunocompromised rodent is an NSG or an NRG mouse engrafted with canine immune cells, and wherein the parasitic worm is *Dirofilaria immitis.*

10. One or more filaricidal compound(s) identified by the method of any one of claims 1 to 9, selected from the group consisting of 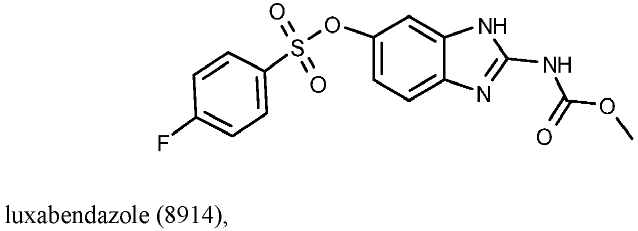 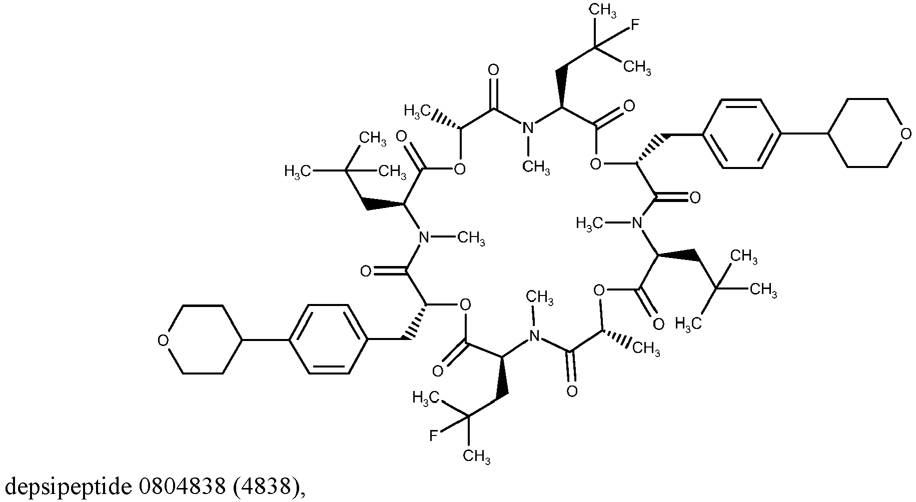 and 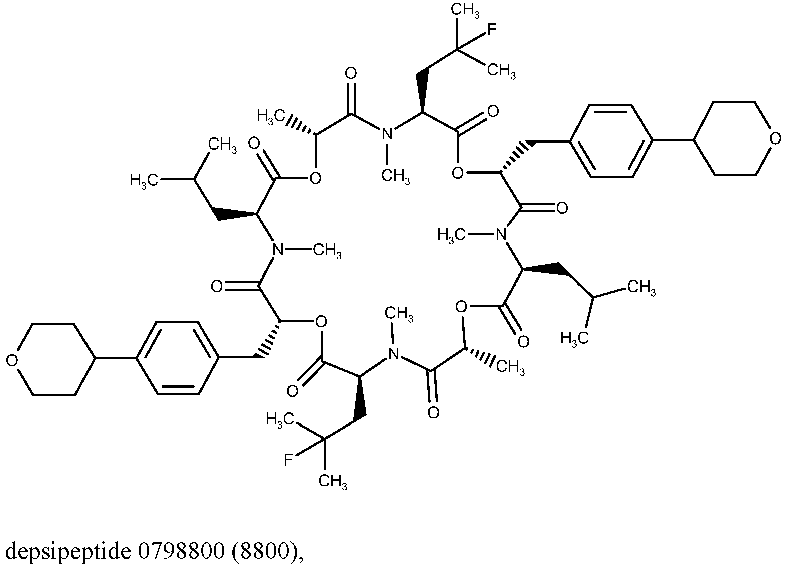 for use in a method of treating filariasis in a subject in need thereof, wherein the filariasis is dirofilariasis.

11. An immunocompromised rodent infected with one or more adult parasitic worms,
wherein the parasitic worm is a nematode and wherein the parasitic worm is *Dirofilaria immitis, Strongyloides stercoralis* or *Haemonchus contortus*
and the rodent is an NOD scid IL2 receptor gamma chain knockout mouse (NSG) (NOD-SCID-IL2Ry -/-) or an NRG (NOD-Rag1^{null}IL2rg^{null}) immunodeficient mouse.

12. The immunocompromised rodent of claim 11, wherein the immunocompromised rodent is an NSG mouse or an NRG mouse, and wherein the parasitic worm is *Dirofilaria immitis;* or
the immunocompromised rodent is an NSG mouse or an NRG mouse comprising engrafted canine immune cells, and wherein the parasitic worm is *Dirofilaria immitis.*

13. Use of an immunocompromised rodent of claim 11 or 12 for modeling complete infection of a parasitic worm outside of the worm's definitive host or for characterizing changes in sensitivity to a parasiticidal or filaricidal agent during parasitic or filarial worm infection.

14. Use of an immunocompromised rodent for modeling complete infection of a parasitic worm outside of the worm's definitive host, wherein:
the rodent is an NOD scid IL2 receptor gamma chain knockout mouse (NSG) (NOD-SCID-IL2Ry -/-) or an NRG (NOD-Rag1^{null}IL2rg^{null}) immunodeficient mouse and the parasitic worm is a nematode and the parasitic worm is *Dirofilaria immitis, Strongyloides stercoralis* or *Haemonchus contortus.*

## Patentansprüche

1. Ein Verfahren zur Bestimmung der Wirksamkeit einer Verbindung oder einer Kombination von Verbindungen zur Behandlung oder Vorbeugung einer parasitischen Wurminfektion, umfassend:
(a) Infizieren eines immunsupprimierten Nagetiers mit einer bestimmten Anzahl an parasitischen Wurmlarven;
(b) Behandeln des infizierten Nagetiers mit der zu testenden Verbindung oder Kombination an Verbindungen; und
(c) Nekropsieren des Nagetiers und Bestimmen der Anzahl an parasitischen Würmern, gegebenenfalls adulten parasitischen Würmern, welche in einem Nagetiergewebe vorhanden sind;
wobei das Nagetier eine NOD scid IL2 Rezeptorgammaketten-Knockout-Maus (NSG) (NOD-SCID-IL2Ry -/-) oder eine NRG (NOD-Rag1^{null}IL2rg^{null}) immungeschwächte Maus ist; und wobei der parasitische Wurm ein Nematode ist und der parasitische Wurm *Dirofilaria immitis, Strongyloides stercoralis* oder *Haemonchus contortus* ist.

2. Das Verfahren gemäß Anspruch 1, ferner umfassend:
(d) Infizieren eines Kontroll-immunsupprimierten Nagetiers mit einer bestimmten Anzahl an parasitischen Wurmlarven;
(c) Nekropsieren des Kontroll-immunsupprimierten Nagetiers und Bestimmen der Anzahl an parasitischen Würmern, gegebenenfalls adulten parasitischen Würmern, welche in einem Nagetiergewebe vorhanden sind; und
(f) Vergleichen der Anzahl der Würmer in dem Nagetiergewebe aus Schritt (c) mit der Anzahl der Würmer in dem Nagetiergewebe aus Schritt (e).

3. Ein Verfahren zur Bestimmung der Anzahl der parasitischen Würmer in einer oder mehreren isolierten Gewebeprobe(n) eines immunsupprimierten Nagetiers, vorzugsweise einem nekropsierten immunsupprimierten Nagetier, welches mit einer bestimmten Anzahl an parasitischen Wurmlarven infiziert wurde und mit einer zu testenden Verbindung oder Kombination an Verbindungen behandelt wurde, wobei das Verfahren das Bestimmen der Anzahl der parasitischen Würmer, gegebenenfalls adulten parasitischen Würmern, welche in der/den isolierten Gewebeprobe(n) vorhanden ist, umfasst; wobei der parasitische Wurm ein Nematode ist und der parasitische Wurm *Dirofilaria immitis, Strongyloides stercoralis* oder *Haemonchus contortus* ist, und wobei das Nagetier eine NOD scid IL2 Rezeptorgammaketten-Knockout-Maus (NSG) (NOD-SCID-IL2Ry -/-) oder eine NRG (NOD-Rag1^{null}IL2rg^{null}) immungeschwächte Maus ist.

4. Das Verfahren gemäß Anspruch 3, ferner umfassend das Bestimmen der Anzahl an parasitischen Würmern in einer oder mehreren isolierten Gewebeprobe(n) eines unbehandelten immunsupprimierten Nagetiers, vorzugsweise eines nekropsierten immunsupprimierten Nagetiers, welches mit einer bestimmten Anzahl an parasitischen Wurmlarven infiziert wurde, wobei das Verfahren das Bestimmen der Anzahl der parasitischen Würmer, gegebenenfalls adulten parasitischen Würmer, welche in der/den isolierten Gewebeprobe(n) des unbehandelten immunsupprimierten Nagetiers vorhanden sind, und Vergleichen der Anzahl der parasitischen Würmer, welche in der/den isolierten Gewebeprobe(n) des unbehandelten immunsupprimierten Nagetiers vorhanden sind, mit der Anzahl der parasitischen Würmer, welche in der/den isolierten Gewebeprobe(n) des behandelten immunsupprimierten Nagetiers vorhanden sind.

5. Das Verfahren gemäß einem der Ansprüche 1 bis 4, wobei die parasitischen Wurmlarven ausgewählt sind aus der Gruppe bestehend aus Microfilariae, L3-Larven und L4-Larven.

6. Das Verfahren gemäß einem der Ansprüche 1 bis 5, wobei das immunsupprimierte Nagetier keine transplantierten Immunzellen von einer anderen Spezies umfasst.

7. Das Verfahren gemäß einem der Ansprüche 1 bis 6, wobei das immunsupprimierte Nagetier transplantierte Immunzellen von einem Endwirt des parasitischen Wurms umfasst, gegebenenfalls wobei die transplantierten Immunzellen Immunzellen von einem Hund sind.

8. Das Verfahren gemäß einem der Ansprüche 1 bis 7, ferner umfassend das Vergleichen der parasitiziden Wirksamkeit der Verbindung oder Kombination von Verbindungen mit der parasitiziden Wirksamkeit einer makrozyklischen Lakton-(ML)-parasitiziden Verbindung, wobei die ML-Verbindung vorzugsweise ausgewählt ist aus Avermectin, Ivermectin, Doramectin, Abamectin, Milbemycin und Moxidectin, und/oder einer Depsipeptid-parasitiziden Verbindung, wobei die Depsipeptid-parasitizide Verbindung vorzugsweise Emodepsid ist.

9. Das Verfahren gemäß Anspruch 1 oder 3, wobei das immunsupprimierte Nagetier eine NSG- oder eine NRG-Maus ist und wobei der parasitische Wurm *Dirofilaria immitis* ist; oder wobei das immunsupprimierte Nagetier eine NSG- oder eine NRG-Maus ist, welcher Immunzellen vom Hund transplantiert wurde, und wobei der parasitische Wurm *Dirofilaria immitis* ist.

10. Eine oder mehrere filarizide Verbindung(en), welche durch das Verfahren gemäß einem der Ansprüche 1 bis 9 identifiziert wurde(n), ausgewählt aus der Gruppe bestehend aus 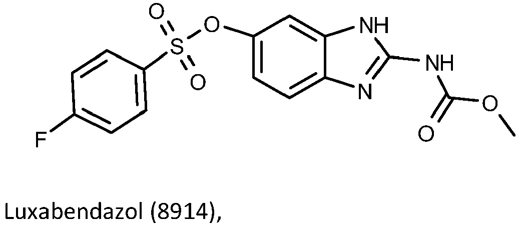 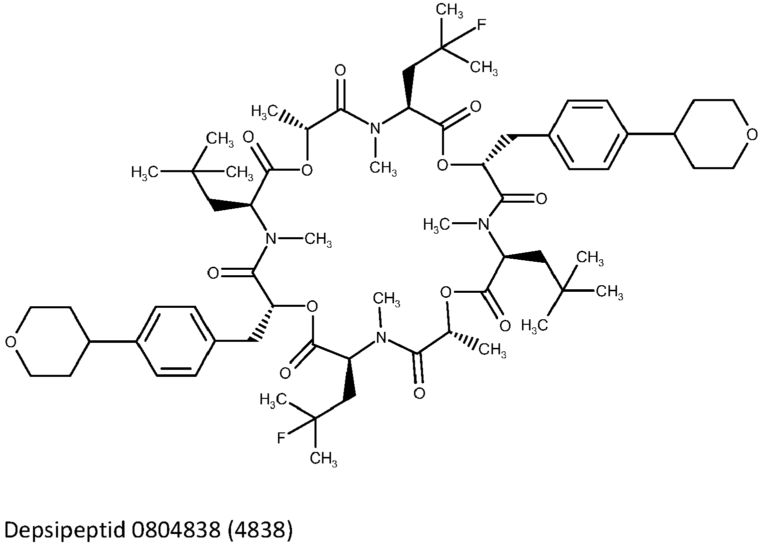 und 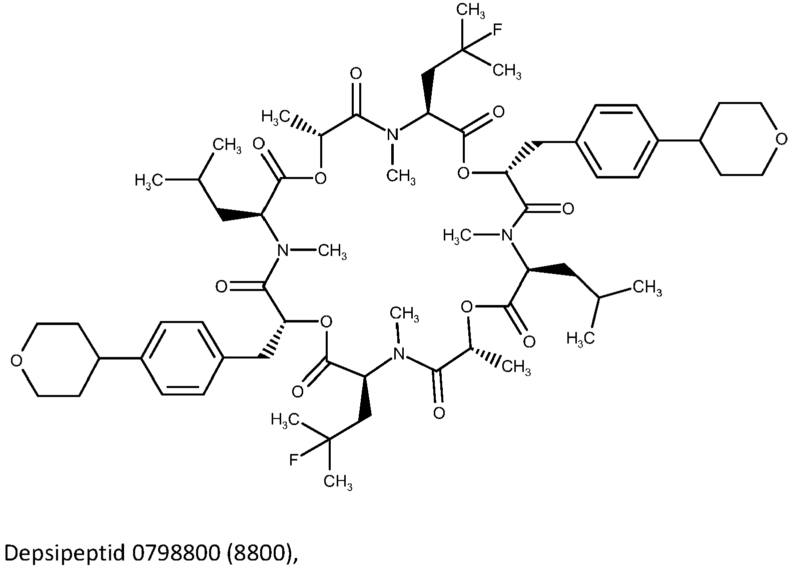 zur Verwendung bei einem Verfahren zur Behandlung von Filariose bei einem Subjekt, welches dieser bedarf, wobei die Filariose Dirofilariose ist.

11. Ein immunsupprimiertes Nagetier, welches mit einem oder mehreren adulten parasitischen Würmern infiziert wurde,
wobei der parasitische Wurm ein Nematode ist und wobei der parasitische Wurm *Dirofilaria immitis, Strongyloides stercoralis* oder *Haemonchus contortus* ist,
und wobei das Nagetier eine NOD scid IL2 Rezeptorgammaketten-Knockout-Maus (NSG) (NOD-SCID-IL2Ry -/-) oder eine NRG (NOD-Rag1^{null}IL2rg^{null}) immungeschwächte Maus ist.

12. Das immunsupprimierte Nagetier gemäß Anspruch 11, wobei das immunsupprimierte Nagetier eine NSG-Maus oder eine NRG-Maus ist und wobei der parasitische Wurm *Dirofilaria immitis* ist; oder
wobei das immunsupprimierte Nagetier eine NSG-Maus oder eine NRG-Maus ist, welche transplantierte Immunzellen von einem Hund umfasst, und wobei der parasitische Wurm *Dirofilaria immitis* ist.

13. Verwendung eines immunsupprimierten Nagetiers gemäß Anspruch 11 oder 12 zur Erstellung eines Modells einer vollständigen Infektion mit einem parasitischen Wurm außerhalb des Endwirts des Wurmes oder zur Charakterisierung von Veränderungen der Empfindlichkeit gegenüber einem parasitiziden oder filariziden Mittel während einer parasitären Wurminfektion oder einer Infektion mit Fadenwürmern.

14. Verwendung eines immunsupprimierten Nagetiers zur Erstellung eines Modells einer vollständigen Infektion mit einem parasitischen Wurm außerhalb des Endwirts des Wurmes, wobei:
das Nagetier eine NOD scid IL2 Rezeptorgammaketten-Knockout-Maus (NSG) (NOD-SCID-IL2Ry -/-) oder eine NRG (NOD-Rag1^{null}IL2rg^{null}) immungeschwächte Maus ist und wobei der parasitische Wurm ein Nematode ist und der parasitische Wurm *Dirofilaria immitis*, *Strongyloides stercoralis* oder *Haemonchus contortus* ist.

## Revendications

1. Procédé de détermination de l'efficacité d'un composé ou d'une combinaison de composés pour traiter ou prévenir une infection par un ver parasite, comprenant:
(a) l'infection d'un rongeur immunodéprimé avec un nombre spécifiques de larves de vers parasites;
(b) le traitement du rongeur infecté avec le composé ou la combinaison de composés à tester; et
(c) la nécropsie du rongeur et la détermination du nombre de vers parasites, éventuellement de vers parasites adultes, présents dans un tissu de rongeur;
où le rongeur est une souris knock-out pour la chaîne gamma de récepteur d'IL2 NOD scid (NSG) (NOD-SCID-IL2Ry -/-) ou une souris immunodéficiente NRG (NOD-Rag1^{null}IL2rg^{null}); et où le ver parasite est un nématode et le ver parasite *est Dirofilaria immitis, Strongyloides stercoralis* ou *Haemonchus contortus.*

2. Procédé selon la revendication 1, comprenant en outre:
(d) l'infection d'un rongeur immunodéprimé témoin avec un nombre spécifique de larves de vers parasites;
(e) la nécropsie du rongeur immunodéprimé témoin et la détermination du nombre de vers, éventuellement des vers parasites adultes, présents dans un tissu de rongeur; et
(f) la comparaison du nombre de vers dans le tissu de rongeur à l'étape (c) avec le nombre de vers dans le tissu de rongeur à l'étape (e).

3. Procédé de détermination du nombre de vers parasites dans un ou plusieurs échantillons de tissu isolés d'un rongeur immunodéprimé, de préférence un rongeur immunodéprimé nécropsié, infecté avec un nombre spécifique de larves de vers parasites et traité avec un composé ou une combinaison de composés à tester, où le procédé comprend la détermination du nombre de vers parasites, éventuellement de vers parasites adultes, présents dans le ou les échantillons de tissu isolés; où le ver parasite est un nématode et le ver parasite est *Dirofilaria immitis, Strongyloides stercoralis* ou *Haemonchus contortus* et où le rongeur est une souris knock-out pour la chaîne gamma de récepteur d'IL2 NOD scid (NSG) (NOD-SCID-IL2Ry -/-) ou une souris immunodéficiente NRG (NOD-Rag1^{null}IL2rg^{null}).

4. Procédé selon la revendication 3, comprenant en outre la détermination du nombre de vers parasites dans un ou plusieurs échantillons de tissu isolés d'un rongeur immunodéprimé non traité, de préférence un rongeur immunodéprimé nécropsié, infecté avec un nombre spécifique de larves de vers parasites, où le procédé comprend la détermination du nombre de vers parasites, éventuellement de vers parasites adultes, présents dans le ou les échantillons de tissu isolés provenant du rongeur immunodéprimé non traité, et la comparaison du nombre de vers parasites présents dans le ou les échantillons de tissu isolés provenant du rongeur immunodéprimé non traité avec le nombre de vers parasites présents dans le ou les échantillons de tissus isolés provenant du rongeur immunodéprimé traité.

5. Procédé selon l'une quelconque des revendications 1 à 4, où les larves de vers parasites sont choisies dans le groupe consistant en les microfilaires, les larves L3 et les larves L4.

6. Procédé selon l'une quelconque des revendications 1 à 5, où le rongeur immunodéprimé ne comprend pas de cellules immunitaires greffées provenant d'une espèce différente.

7. Procédé selon l'une quelconque des revendications 1 à 6, où le rongeur immunodéprimé comprend des cellules immunitaires greffées provenant d'un hôte définitif du ver parasite, éventuellement où les cellules immunitaires greffées sont des cellules immunitaires canines.

8. Procédé selon l'une quelconque des revendications 1 à 7, comprenant en outre la comparaison de l'efficacité parasiticide du composé ou de la combinaison de composés par rapport à l'efficacité parasiticide de l'un ou des deux d'un composé parasiticide lactone macrocyclique (ML), de préférence où le composé ML est choisi parmi l'avermectine, l'ivermectine, la doramectine, l'abamectine, la milbémycine et la moxidectine, et/ou d'un composé parasiticide depsipeptide, de préférence où le composé parasiticide depsipeptide est l'emodepside.

9. Procédé selon la revendication 1 ou 3, où le rongeur immunodéprimé est une souris NSG ou NRG, et où le ver parasite est *Dirofilaria immitis*; ou bien où le rongeur immunodéprimé est une souris NSG ou NRG greffée avec des cellules immunitaires canines, et où le ver parasite est *Dirofilaria immitis.*

10. Un ou plusieurs composés filaricides identifiés par le procédé selon l'une quelconque des revendications 1 à 9, choisis dans le groupe consistant en 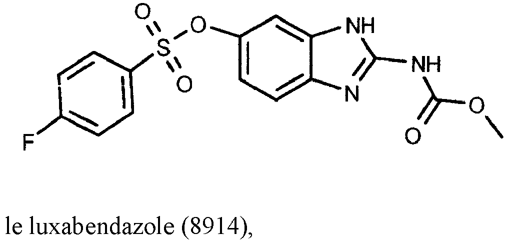 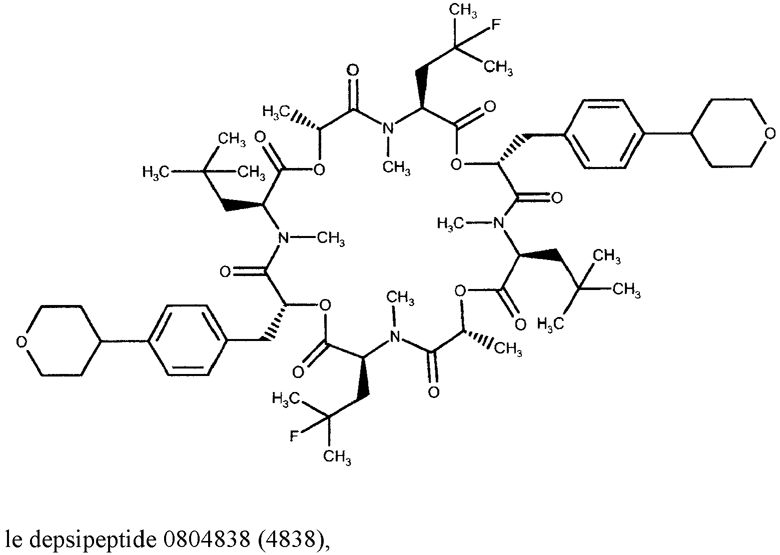 et 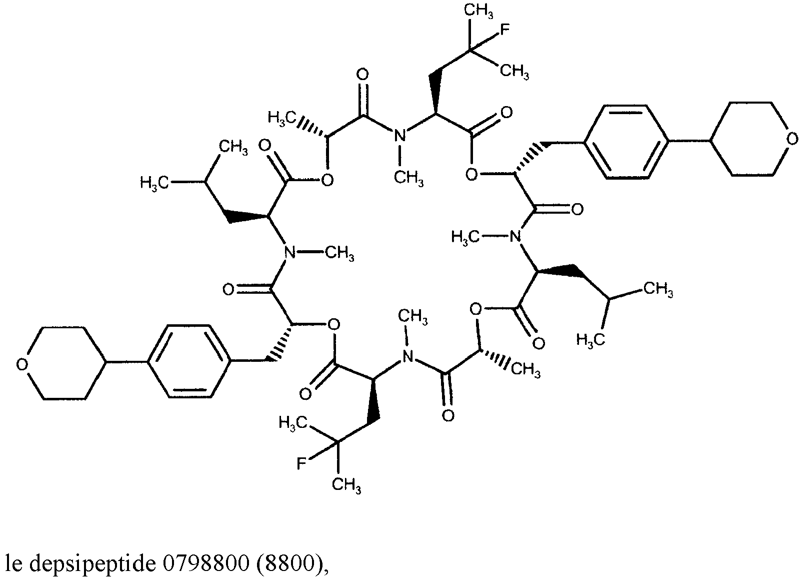 destinés à être utilisés dans un procédé de traitement de la filariose chez un sujet en ayant besoin, où la filariose est la dirofilariose.

11. Rongeur immunodéprimé infecté par un ou plusieurs vers parasites adultes,
où le ver parasite est un nématode et où le ver parasite est *Dirofilaria immitis*, *Strongyloides stercoralis* ou *Haemonchus contortus*
et le rongeur est une souris knock-out pour la chaîne gamma de récepteur d'IL2 NOD scid (NSG) (NOD-SCID-IL2Ry -/-) ou une souris immunodéficiente NRG (NOD-Rag1^{null}IL2rg^{null}).

12. Rongeur immunodéprimé selon la revendication 11, où
le rongeur immunodéprimé est une souris NSG ou une souris NRG, et où le ver parasite est *Dirofilaria immitis*; ou
le rongeur immunodéprimé est une souris NSG ou une souris NRG comprenant des cellules immunitaires canines greffées, et où le ver parasite est *Dirofilaria immitis.*

13. Utilisation d'un rongeur immunodéprimé selon la revendication 11 ou 12 pour modéliser l'infection complète d'un ver parasite à l'extérieur de l'hôte définitif du ver ou pour caractériser les changements de sensibilité à un agent parasiticide ou filaricide lors d'une infection par un ver parasite ou filarien.

14. Utilisation d'un rongeur immunodéprimé pour modéliser l'infection complète d'un ver parasite à l'extérieur de l'hôte définitif du ver, où:
le rongeur est une souris knock-out pour la chaîne gamma de récepteur d'IL2 NOD scid (NSG) (NOD-SCID-IL2Ry -/-) ou une souris immunodéficiente NRG (NOD-Rag1^{null}IL2rg^{null}) et le ver parasite est un nématode et le ver parasite *est Dirofilaria immitis*, *Strongyloides stercoralis* ou *Haemonchus contortus.*
